(19) Europäisches Patentamt
European Patent Office
Office européen des brevets

(11) Publication number: **0 534 511 A1**

# (12) EUROPEAN PATENT APPLICATION

(21) Application number: **92202447.6**

(22) Date of filing: **08.08.92**

(51) Int. Cl.5: **C07D 207/12**, C07D 307/20, C07D 309/10, C07D 333/32, C07D 333/48, C07D 335/02, C07D 407/12, C07D 409/12, A61K 31/35, A61K 31/38, A61K 31/41

(30) Priority: **16.08.91 US 746686**

(43) Date of publication of application: **31.03.93 Bulletin 93/13**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Ghosh, Arun, K.**
**853 Jackson Street**
**Lansdale, PA 19446(US)**
Inventor: **Huff, Joel R.**
**825 Surrey Drive**
**Gwynedd Valley, PA 19437(US)**
Inventor: **Thompson, Wayne J.**
**2291 Locust Drive**
**Lansdale, PA 19446(US)**
Inventor: **Lyle, Terry A.**
**570 Camp WaWa Road**
**Lederach, PA 19450(US)**
Inventor: **Hungate, Randall W.**
**1925 Rampart Lane**
**Lansdale, PA 19446(US)**
Inventor: **McKee, Sean P.**
**437 Grove Street**
**Bridgeport, PA 19405(US)**
Inventor: **Lee, Hee Yoon**
**190 Logan Drive**
**Hatfield, PA 19440(US)**

(74) Representative: **Barrett-Major, Julie Diane et al**
**Merck & Co., Inc. European Patent Department Terlings Park Eastwick Road**
**Harlow Essex CM20 2OR (GB)**

## (54) HIV protease inhibitors useful for the treatment of aids.

(57) Compounds of the form,

A-G-J

wherein A is an amine protecting group or urethane, G a dipeptide isostere and J a small terminal group are described. They are distinguished by tetrahydrofuryl or tetrahydropyranyl substituents, and the like. These compounds are useful in the inhibition of HIV protease, the prevention or treatment of infection by HIV and the treatment of AIDS, either as compounds, pharmaceutically acceptable salts, pharmaceutical composition ingredients, whether or not in combination with other antivirals, immunomodulators, antibiotics or vaccines. Methods of treating AIDS and methods of preventing or treating infection by HIV are also described.

EP 0 534 511 A1

The present invention is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds and to a method of use of the present compounds and other agents for the treatment of AIDS & viral infection by HIV.

## BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Inhibition of this processing prevents the production of normally infectious virus. For example, Kohl, N.E. et al., Proc. Nat'l Acad. Sci. 85, 4686 (1988) demonstrated that genetic inactivation of the HIV encoded protease resulted in the production of immature, non-infectious virus particles. These results indicate that inhibition of the HIV protease represents a viable method for the treatment of AIDS and the prevention or treatment of infection by HIV.

The nucleotide sequence of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M.D. et al., Science, 231, 1567 (1986); Pearl, L.H. et al., Nature 329, 351 (1987)]. Applicants demonstrate that the compounds of this invention are inhibitors of HIV protease. Compounds in this invention are distinguished by N-terminal tetrahydrofuryl or tetrahydropyranyl substitutents, and the like

## BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

## ABBREVIATIONS

| Designation | Activating Group |
|---|---|
| HBT(HOBT or HOBt) | 1-hydroxybenzotriazole hydrate |
| | **Reagent** |
| $(BOC)_2O$ | di-t-butyl dicarbonate |
| $BF_3 \bullet OET_2$ | boron trifluoride etherate |
| | **Coupling Reagents** |
| EDC | 1-ethyl-3-(3-dimethyl- |

| | |
|---|---|
| | aminopropyl) carbodiimide hydrochloride |
| MCPBA | m-Chloroperbenzoic acid |
| TBDMS | t-Butyl-dimethylsilyl |

DETAILED DESCRIPTION OF THE INVENTION AND PREFERRED EMBODIMENTS

This invention is concerned with compounds of formula I, combinations thereof, or pharmaceutically acceptable salts thereof, in the inhibition of HIV protease, the prevention or treatment of infection by HIV and in the treatment of the resulting acquired immune deficiency syndrome (AIDS). Compounds of formula I are defined as follows:

A-G-J I,

wherein A is:

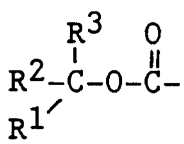 wherein $R^1$, is

a) H, or
b) $C_{1-6}$ alkyl unsubstituted or substituted with one or more of
  i) OH
  ii) alkoxy; and

$R^2$ and $R^3$ are joined to form a 5-7 membered heterocyclic ring, or 7-10 membered bicyclic heterocyclic ring, saturated or unsaturated, unsubstituted or substituted with one or more of $R^4$ wherein

$R^4$ is
$R^5$-$(CH_2)_n$-, $R^5(CH_2)_nO$-,
$R^5$-$(CH_2)_nS$-, $R^5(CH_2)_nS(O)$-, or
$R^5$-$(CH_2)SO_2$- wherein

$R^5$ is
aryl, heterocycle,
$C_{1-4}$ alkyl or H and n is 0-5.

G is

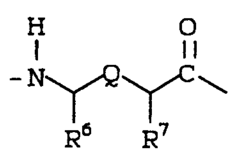 or 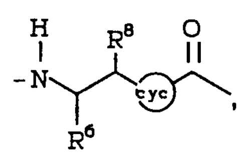,

$R^6$ and $R^7$ are independently,
a) $-(CH_2)_n-R^9$,
b) $C_{1-4}$alkenyl $-R^9$;
wherein n is 0-5

$R^9$ is
a) hydrogen;
b) $C_{1-4}$ alkyl;
c) $C_5$-$C_{10}$ cycloalkyl, unsubstituted or substituted one or more times with hydroxy;

d) $C_6$-$C_{10}$ aryl, unsubstituted or substituted one or more times with;
  i) halo,
  ii) $C_{1-4}$alkyl, unsubstituted or substituted once with hydroxy,
  iii) $C_{1-3}$ alkoxy, unsubstituted or substituted once with hydroxy,
  iv) hydroxy;
e) monocyclic or bicyclic heterocycle containing from 1 to 3 heteroatoms chosen from N,O,S and which is unsubstituted or substituted with one or more of
  i) halo,
  ii) $C_{1-4}$ alkyl,
  iii) $C_{1-3}$ alkoxy;

$R^8$ is -OH or -NHR$^{10}$, wherein $R^{10}$ is -H,

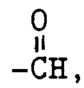

or -$C_{1-4}$- alkyl;

Q is

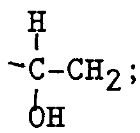 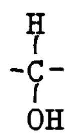 ; or 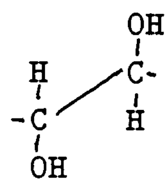

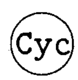

is
1) $C_{3-7}$ cycloalkyl either unsubstituted or substituted with one or more of
  a) $C_{1-4}$ alkyl,
  b) hydroxy,
  c) -$NR_2$, wherein R is H or $C_{1-4}$ alkyl;
  d) -COOR,
  e) -CONHR,
  f) -$NHSO_2R$,
  g)

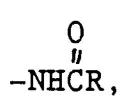

  h) aryl,
  i) aryl substituted with $C_{1-4}$alkyl,
  j) heterocycle, or
  k) heterocycle substituted with $C_{1-4}$alkyl;

J is

a) NHR$^{11}$ wherein $R^{11}$ is a 5-7 membered heterocyclic ring or, 7-10 membered bicyclic heterocyclic ring which is saturated or unsaturated, such as isochroman, chroman, isothiochroman, thiochroman, benzimidazole, benzothiopyran, oxobenzothiopyran, benzopyran, benzothiopyranylsulfone, benzothiopyranylsulfoxide, the ring or rings being unsubstituted or substituted with one or more of

i) halo,
ii) -OR, wherein R is H, $C_{1-4}$alkyl, or $C_{1-4}$alkenyl,
iii)

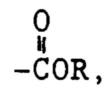

iv)

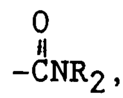

v) $-CH_2NR_2$,
vi) $-SO_2NR_2$,
vii) $-NR_2$,
viii)

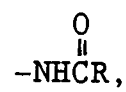

xi) $C_{1-4}$ alkyl,
x) phenyl
xi) $-CF_3$,
xii)

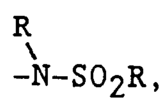

xiii) phenyl $C_{1-4}$ alkyl,
xiv) $-OP(O)(OR_x)_2$ wherein $R_x$ is H or aryl,
xv)

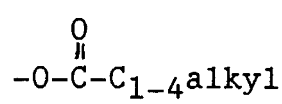

substituted with one or more of amine or quaternary amine, or $-OP(O)(OR_x)_2$, or $-O[-(CH_2)_mO]_n-R$, wherein m is 2-5, n is 0-5; or
xvi)

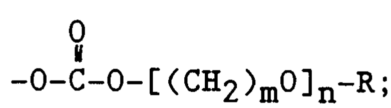

b) A 5 to 7 membered carbocyclic or 7-10 membered bicyclic carbocyclic ring which is either saturated or unsaturated, such as cyclopentane, cyclohexane, indane, norbornane, or naphthalene, the carbocyclic ring being unsubstituted or substituted with one or more of
i) halo,
ii) -OR, or $-CH_2OR$, wherein R is H or $C_{1-4}$ alkyl,
iii)

$$-\overset{O}{\overset{\|}{C}}OR^{12},$$

wherein $R^{12}$ is H,
$-(CH_2)_n-NR_2$, $C_{1-16}$alkyl, pyridine, $-(CH_2)_nNR-(CH_2)_n-NR_2$,

$$-(CH_2)_n-\overset{O}{\overset{\|}{C}}-OR,$$

$-[(CH_2)_mO]_n-R$, quinuclidiniumyl substituted with R, piperazine- $C_{1-4}$alkyl-benzyl substituted once or more with R, or morpholinoalkyl- benzyl,
iv)

$$-\underset{\underset{O}{\|}}{C}NR_2,$$

v) $-CH_2NR_2$,
vi) $-SO_2NR_2$,
vii) $-NR_2$,
viii)

$$-NH\overset{O}{\overset{\|}{C}}R,$$

xi) $C_{1-4}$ alkyl,
x) phenyl,
xi) $-CF_3$,
xii)

$$-\overset{R}{\overset{|}{N}}-SO_2R,$$

xiii) $-OP(O)(OR_x)_2$ wherein $R_x$ is H or aryl,
xiv)

$$-O-\overset{O}{\overset{\|}{C}}-C_{1-4}alkyl$$

substituted with one or more of amine or quaternary amine, $-OP(O)(OR_x)_2$, or $-O-[-(CH_2)_mO]_n-R$, or
xv)

$$-O-\overset{O}{\overset{\|}{C}}-O-[(CH_2)_mO]_n-R;$$

or pharmaceutically acceptable salts or esters thereof.

In a preferred embodiment of this invention,

A is tetrahydrofuranyloxycarbonyl or tetrahydropyranyloxycarbonyl, unsubstituted or substituted with $R_4$; and

G is

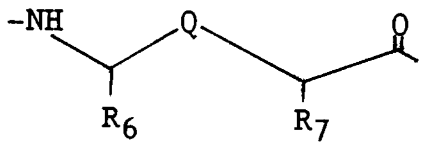

.

In a more preferred embodiment of this invention,

Q is

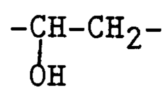

.

It is most preferred that J is NH-$R^{11}$ wherein $R^{11}$ is a 7 to 10 membered bicyclic carbocyclic or heterocyclic ring which is either saturated or unsaturated and is substituted with hydroxyl, particularly $R^{11}$ as indanyl substituted once or twice with hydroxyl.

Novel compounds of the present invention include, but are not limited to, the following:

N-(2(R)-hydroxy-1(S)-indanyl)-5(S)-(3(S)-tetrahydrofuranyloxycarbonylamino)-4(S)-hydroxy-6-phenyl-2(R)-(4-(2-hydroxyethoxy)phenyl)methyl hexanamide,

N-(2(R)-hydroxy-1(S)-indanyl)-5(S)-(3(S)-tetrahydrofuranyloxycarbonylamino)-4(S)-hydroxy-6-phenyl-2(R)-phenylmethyl hexanamide,

N-(2(R)-hydroxy-1(S)-indanyl)-5(S)-(3(S)-tetrahydrofuranyloxycarbonylamino)-4(S)-hydroxy-6-phenyl-2(R)-methyl hexanamide,

N-(2(R)-hydroxy-1(S)-indanyl)-5(S)-(3(S)-tetrahydrofuranyloxycarbonylamino)-4(S)-hydroxy-6-phenyl-2(R)-(trans-3-(phenyl)prop-2-en-1-yl)hexanamide,

N-(2(R)-hydroxy-1(S)-indanyl)-5(S)-(3(S)-tetrahydrofuranyloxycarbonylamino)-4(S)-hydroxy-6-phenyl-2(R)-ethyl hexanamide,

N-(2(R)-hydroxy-1(S)-indanyl)-5(S)-(3(S)-tetrahydrofuranyloxycarbonylamino)-4(S)-hydroxy-6-phenyl-2(R)-cyclopropylmethyl hexanamide,

N-(2(R)-hydroxy-1(S)-indanyl)-5(S)-(3(S)-tetrahydrofuranyloxycarbonylamino)-4(S)-hydroxy-6-cyclohexyl-2-(R)-cyclohexylmethyl hexanamide,

N-(2(R)-hydroxy-3(R)-amino-1(S)-indanyl)-5(S)-(3(S)-tetrahydrofuranyloxycarbonylamino)-4(S)-hydroxy-6-cyclohexyl-2(R)-phenylmethyl hexanamide,

N-(2(R)-hydroxy-1(S)-indanyl)-5(S)-(3(S)-tetrahydrofuranyloxycarbonylamino)-4(S)-hydroxy-6-cyclohexyl-2-(R)-phenylmethyl hexanamide,

N-(2(R)-hydroxy-1(S)-indanyl)-5(S)-(3(S)-tetrahydrofuranyloxycarbonylamino)-4(S)-hydroxy-6-cyclohexyl-2-(R)-(4-(2-hydroxyethoxy)phenyl)methyl hexanamide,

N-(2(R)-hydroxy-1(S)-indanyl)-5(S)-(3(S)-tetrahydrofuranyloxycarbonylamino)-4(S)-hydroxy-6-cyclohexyl-2-(R)-(1-methylethyl) hexanamide,

N-(2(R)-hydroxy-1(S)-indanyl)-5(S)-(3(S)-tetrahydrofuranyloxycarbonylamino)-4(S)-hydroxy-7-methyl-2(R)-phenylmethyl octanamide,

N-(2-(R)-hydroxy-5(R)-methyl-1(S)-cyclopentyl)-5(S)-(3(S)-tetrahydrofuranyloxycarbonylamino)-4(S)-hydroxy-6-phenyl-2(R)-(4-(2-hydroxyethoxy)phenyl)-methyl hexanamide,

N-(3,4-dihydro-2,2-dioxo-1H-4(S)-benzo-2-thiopyranyl)-5(S)-(3(S)-tetrahydrofuranyloxycarbonylamino)-4(S)-hydroxy-6-phenyl-2(R)-phenylmethyl hexanamide,

N-(3,4-dihydro-1H-4(S)-benzo-2-thiopyranyl)-5(S)-(3(S)-tetrahydrofuranyloxycarbonylamino)-4(S)-hydroxy-6-phenyl-2(R)-(3-phenyl-prop-2-en-1-yl)methyl hexanamide,

N-(3,4-dihydro-2(R,S)-oxo-1H-4(S)-benzo-2-thiopyranyl)-5(S)-(3(S)-tetrahydrofuranyloxycarbonylamino)-4(S)-hydroxy-6-phenyl-2(R)-(3-phenyl-prop-2-en-1-yl)methyl hexanamide,

N-(2(R)-hydroxy-1(S)-indanyl)-5(S)-(3(R,S)-tetrahydropyranyloxycarbonylamino)-4(S)-hydroxy-6-phenyl-2(R)-(3-phenyl-prop-2-en-1-yl)methyl hexanamide,

N-(1(R)-amino-2(R)-hydroxy-3(S)-indanyl)-5(S)-(3(S)-tetrahydrofuranyloxycarbonylamino)-4(S)-hydroxy-6-cyclohexyl-2(R)-phenylmethyl hexanamide,
N-(3,4-dihydro-2,2-dioxo-1H-4(S)-benzo-2-thiopyranyl)-5(S)-(3(S)-tetrahydrofuranyloxycarbonylamino)-4(S)-hydroxy-6-phenyl-2(R)-(3-phenyl-prop-2-en-1-yl)methyl hexanamide,
N-(2(R)-hydroxy-1(S)-indanyl)-5(S)-(3(S)-1,1-dioxotetrahydrothiofuranyloxycarbonylamino)-4(S)-hydroxy-6-phenyl-2(R)-(3-(phenyl)prop-2-en-1-yl)hexamide,
N-(2(R)-hydroxy-1(S)-indanyl)-5(S)-(3(S)-1,1-dioxotetrahydrothiopyranyloxycarbonylamino)-4(S)-hydroxy-6-phenyl-2(R)-(3-(phenyl-prop-2-en-1-yl) hexanamide,
N-(2(R)-hydroxy-1(S)-indanyl)-5(S)-(3(S)-tetrahydrofuranyloxycarbonylamino)-4(S)-hydroxy-6-methyl-2(R)-(1-methylethyl) heptanamide,
N-(2(R)-hydroxy-1(S)-indanyl)-5(S)-(3(S)-tetrahydrofuranyloxycarbonylamino)-4(S)-hydroxy-6-methyl-2(R)-phenylmethyl heptanamide,
N-(2(R)-hydroxy-1(S)-indanyl)-5(S)-(3(S)-tetrahydrofuranyloxycarbonylamino)-4(S)-hydroxy-6-methyl-2(R)-(3-phenyl-prop-2-en-1-yl)methyl heptanamide,
N-(2(R)-hydroxy-1(S)-indanyl)-5(S)-(3(S)-tetrahydrofuranyloxycarbonylamino)-4(S)-hydroxy-7-methyl-2(R)-(1-methylethyl) octanamide,
N-(2(R)-hydroxy-1(S)-indanyl)-5(S)-(3(S)-tetrahydrofuranyloxycarbonylamino)-4(S)-hydroxy-7-methyl-2(R)-(3-phenyl-prop-2-en-1-yl)methyl octanamide,
N-(2(R)-hydroxy-1(S)-indanyl)-5(S)-(3(R,S)-pyrrolidinyloxycarbonylamino)-4(S)-hydroxy-6-phenyl-2(R)-[4-(2-hydroxyethoxyphenyl)methyl]hexanamide,
N-(2(R)-hydroxy-1(S)-indanyl)-5(S)-(1-benzyloxycarbonyl-3(R,S)-pyrrolidinyloxycarbonylamino)-4(S)-hydroxy-6-phenyl-2(R)-[4-(2-hydroxyethoxyphenyl)-methyl hexanamide,
N-[cis-2(R)-hydroxy-1(S)-indanyl]-5-[3(S)-tetrahydro-2H-pyranyloxycarbonylamino]-4(S)-hydroxy-6-cyclohexyl-2(R)-(3-phenyl-2-propen-1-yl)-hexanamide,
N-[cis-2(S)-hydroxy-1(S)-benzopyranyl]-5-[3(S)-tetrahydrofuranyloxycarbonylamino]-4(S)-hydroxy-2(R)-phenylmethyl pentanamide,
N-[cis-2(S)-hydroxy-1(S)-benzopyranyl]-5-[3(R)-(2-(S)-4-phenylbutyl)tetrahydro-2H-pyranyloxycarbonylamino]-4(S)-hydroxy-2(R)-phenylmethyl pentanamide,
N-[cis-2(S)-hydroxy-1(S)-benzopyranyl]-5-[3(S)-(2-(R)-4-phenylbutyl)tetrahydro-2H-pyranyloxycarbonylamino]-4(S)-hydroxy-2(R)-phenylmethyl pentanamide,
N-[cis-2(S)-hydroxy-1(S)-benzopyranyl]-5-[3(R)-(2-(S)-3-phenylpropyl)tetrahydro-2H-pyranyloxycarbonylamino]-4(S)-hydroxy-2(R)-phenylmethyl pentanamide,
N-[cis-2(S)-hydroxy-1(S)-benzopyranyl]-5-[3(S)-(2-(R)-3-phenylpropyl)tetrahydro-2H-pyranyloxycarbonylamino]-4(S)-hydroxy-2(R)-phenylmethyl pentanamide,
N-[cis-2(R)-hydroxy-1(S)-indanyl]-5(S)-[3'(S)-tetrahydrofuranyloxycarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[3(S)-tetrahydropyranyloxycarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[3(SR)-tetrahydrothiofuranyloxycarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[3(SR)-1-oxo tetrahydrothiofuranyloxycarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[3(SR)-1,1-dioxo-tetrahydrothiofuranyloxycarbonylamino] -4(S)-hydroxy-6-cyclohexyl-2(R)-(3-phenyl-2-propen-1-yl)-hexanamide, N-[cis-2(R)-hydroxy-1(S)-indanyl]-5(S)-[4'-(SR)-tetrahydrothiopyranyloxycarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[4(SR)-1,1-dioxo- tetrahydrothiopyranylcarbonylamino]-4(S)-hydroxy-6-cyclohexyl-2(R)-(3-phenyl-2-propen-1-yl)-hexanamide, N-[cis-2(R)-hydroxy-1(S)-indanyl]-5(S)-[6(R)-1-oxomethoxy-3(S)-tetrahydropyranylcarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[6(S)-methoxy-3(S)-tetrahydropyranyloxycarbonylamino]-4(S)-hydroxy-6-cyclohexyl-2(R)-(3-phenyl-2-propen-1-yl)-hexanamide,
N-[cis-2(R)-hydroxy-1(S)-indanyl]-5(S)-[5(S)-iodomethyl-3(S)-tetrahydrofuranyloxycarbonylamino]-4(S)-hydroxy-6-cyclohexyl-2(R)-(3-phenyl-2-propen-1-yl)-hexanamide,
N-[(2R)-hydroxy-1(S)-indanyl]-5(S)-(3(S)-tetrahydrofuranyloxycarbonylamino)-4(S)-hydroxy-6-cyclohexyl-2-(R)-isopropylhexanamide,
N-[(2R)-hydroxy-1(S)-indanyl ornithine ester]-5(S)-(3(S)-tetrahydrofuranyloxycarbonylamino)-4(S)-hydroxy-6-cyclohexyl-2(R)-isopropyl hexanamide,
N-[(2R)-hydroxy-1(S)-indanyl]-5(S)-(3(S)-tetrahydrofuranyloxycarbonylamino)-4(S)-hydroxy-6-cyclohexyl-2-

(R)-phenylmethyl hexanamide, or
N-[(2R)-hydroxy-1(S)-indanyl ornithine ester]-5(S)-(3(S)-tetrahydrofuranyloxycarbonylamino)-4(S)-hydroxy-6-cyclohexyl-2(R)-phenylmethyl hexanamide,
or pharmaceutically acceptable salts or esters thereof.

Preferred species of the present invention include:

A.

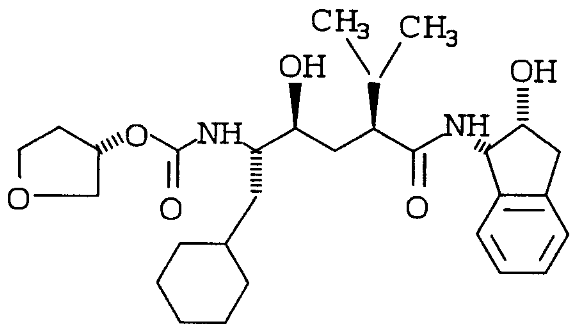

,

N-[(2R)-hydroxy-1(S)-indanyl]-5(S)-(3(S)-tetrahydrofuranyloxycarbonylamino)-4(S)-hydroxy-6-cyclohexyl-2-(R)-isopropyl hexanamide,
and its ornithine ester prodrug:

B:

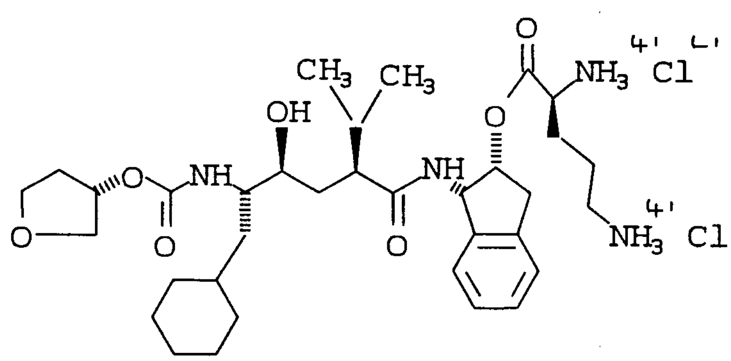

,

N-[(2R)-hydroxy-1(S)-indanyl ornithine ester]-5(S)-(3(S)-tetrahydrofuranyloxycarbonylamino)-4(S)-hydroxy-6-cyclohexyl-2(R)-isopropyl hexanamide.

C.

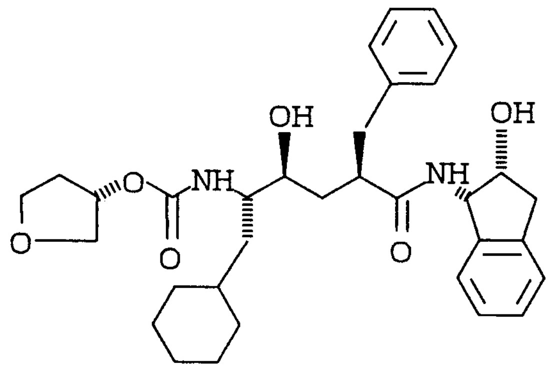

,

N-[(2R)-hydroxy-1(S)-indanyl]-5(S)-(3(S)-tetrahydrofuranyloxycarbonylamino)-4(S)-hydroxy-6-cyclohexyl-2-(R)-phenylmethyl hexamamide,
and its ornithine ester prodrug:

D.

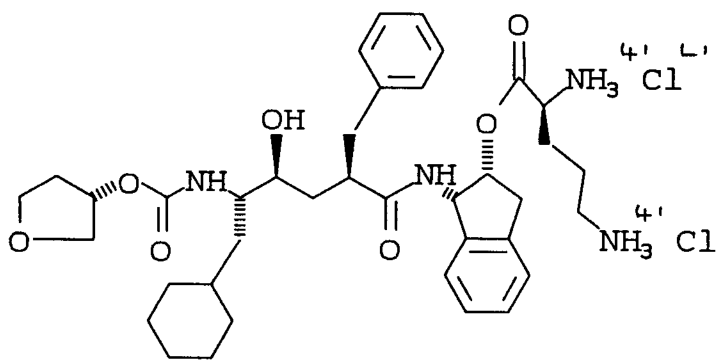

,

N-[(2R-hydroxy-1(S)-indanyl ornithine ester]-5(S)-(3(S)-tetrahydrofuranyloxycarbonylamino)-4(S)-hydroxy-6-cyclohexyl-2(R)-phenylmethyl hexanamide.

In the compounds of the present invention, the A, G and J components and the like may have asymmetric centers and occur as racemates, racemic mixtures and as individual diastereomers or enantiomers, with all isomeric forms being included in the present invention.

When any variable (e.g., aryl, heterocycle, R, $R^1$, $R^2$, $R^3$, $R^4$, $R^7$, $R^9$, $R^{10}$, $R^{11}$, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "cycloalkyl" is intended to include saturated ring groups, such as cyclopropyl, cyclobutyl, cyclopentyl, cyclohexyl (Cyh) and cycloheptyl. "Alkenyl" is intended to include hydrocarbon claims of either a straight or branched configuration and one or more unsaturated carbon-carbon bonds which may occur in any stable point along the chain, such as ethenyl, propenyl, butenyl, pentenyl, and the like. "Halo", as used herein, means fluoro, chloro, bromo and iodo.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring, any of which may be saturated or partially unsaturated.

The term heterocycle or heterocyclic or heterocyclic ring, as used herein except where noted, represents a stable 5- to 7-membered mono- or bicyclic or stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, pyranyl, thienyl, benzothienyl, thiamorpholinyl, thiamorpholinyl sulfoxide, thiamorpholinyl sulfone, and oxadiazolyl.

The pharmaceutically-acceptable salts of the compounds of Formula I (in the form of water- or oil-soluble or dispersible products) include the conventional non-toxic salts or the quaternary ammonium salts of these peptides, which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

Pharmaceutically-acceptable esters of the compounds of Formula I encompasses those readily apparent to the skilled antisan, for example, $C_{1-4}$ alkyl esters.

Pharmaceutically acceptable prodrugs of the compounds of the present inventors include conventional esters or salts, such as, N-acetylglycinate, acetate(ester) and octadecanoate(salt) or acetate(ester) and stearate(salt), 1-acetoxyethyl, benzenesulfonate, butyrate(ester) and propionate(ester), camphorsulfonate, hexanoate, p-cholorobenzenesulfonate, 4-methylbicyclo[2.2.2]oct-2-ene-1-carboxylate, cyclopentanepropionate, diethanolamine, ethylenediaminetetraacetate, 1,2-ethanedisulfonate, heptanoate, tert-butylamine, propionate lauryl sulfate, ethanesulfonate, glucoheptonate, o-(4-hydroxybenzoyl)benzoate, monohydrochloride hemiethanolate hemihydrate, 2-hydroxyethanesulfonate, N-methylglucamine, methanesulfonate, 2-naphthalenesulfonate, ethanolamine, 4,4'-methylenebis-(3-hydroxy-2-naphthoate), 3-phenylpropionate, trimethylacetate, 1-(2-methoxy-2-methyl-1- oxopropoxy)ethyl or 1-[(2-methoxy-2- methylpropionyl)-oxy]ethyl, (2,2-dimethyl-1- oxopropoxy)methyl or (pivaloyloxy)methyl, tertiary butyl acetate, p-toluenesulfonate, triethanolamine, methylphenylmethylene, 4,4'-diaminostilbene-2,2'-disulfonate, 3,7-di-tert-butyl-1,5-naphthalenedisulfonate, p-carboxybenzenesulfonate, [(6-hydroxy-4-methyl-2-oxo-2H-1-benzopyran-7-yl)oxy]-acetate, 6,7-dihydroxycoumarin-4-methanesulfonate, 2-(dimethylamino)ethyl, decyl, 2,6-di-tert-butyl-1,5-naphthalenedisulfonate, 2,6-di-tert-butyl-1-naphthalenesulfonate, 2-(2-hydroxyethoxy)ethyl, 4,4'-methylenebis(3-hydroxy-2-naphthoate), o-[(2'-hydroxy-4-biphenylyl)carbonyl]benzoate, n-dodecyl, n-dodecylsulfate, 3,4,5-trimethoxybenzoate, 3-methoxy-2-naphthoate, methylsulfate, 1,5-naphthalenedisulfonate, 2-oxoglutarate, (pivaloyloxy)methyl, 1-[(isopropoxycarbonyl)oxy]ethyl, stearoyl-glycolate, 2-thiophenecarboxylate, 8-chlorotheophyllinate, 1,2,3,6-tetrahydro-1,3-dimethyl-2,6-dioxopurine-7-propanesulfonate; 1,2,3,6-tetrahydro-1,3- dimethyl-2,6-dioxopurine-7-ethanesulfonate, 2,4,5-trichlorophenolate, or [2-(2-ethoxyethoxy)- ethoxy]acetate. Other prodrugs include amino acid esters, and preferably basic amino acid ester prodrugs, such as those of ornithine, lysine, arginine, and the like.

HIV protease inhibitors of Formula I may be prepared in accordance with well-known procedures for preparing peptide analogs from their constituent amino acids or analogs thereof. In general, once the G substituent is made, the rest of the synthesis follows the principle of amide bond formation by the coupling methods of either solution-phase or solid-phase peptide synthesis. The addition and removal of one or more protecting groups is also typical practice.

Structures and abbreviations for the G components of the inhibitors of the present invention include:

1. 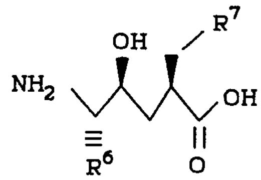 ,

a hydroxyethylene dipeptide isostere prepared, for example, by an intermediate lactone according to Evans, B.E. et al J. Org. Chem. 50, 4615(1985) or Kempf, D.J. J. Org. Chem. 51, 3921(1986). Synthetic routes to similar peptide bond isosteres, such as

2. 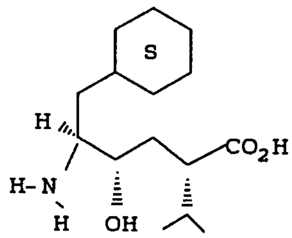

Cal[CH(OH)$CH_2$]Val,

are readily available. The intermediate BOC-Cal[CH(OH)$CH_2$]Val-OH lactone is obtained from intermediates prepared using methods described by P. Buhlmayer et al, in Published European Patent Application 184,550-A2. Other synthetic routes to peptide bond isosteres of like character are described in the following:

a. Szelke et al, in Peptides, Structure and Function. Proceedings of the Eighth American Peptide Symp- (ed. V.J. Hruby and D.H. Rich) pp. 579-82, Pierce Chemical Co., Rockford, IL;

b. D.T. Pals et al in European Patent Appln. 173,481-A2;

c. A.H. Fray et al, J. Org. Chem., 51, 4828-4833 (1986); and

d. M. Szelke et al, PCT Int. Appl. WO 84 03,044;

Structures and abbreviations for other G components of the inhibitors of the present invention include:

3. 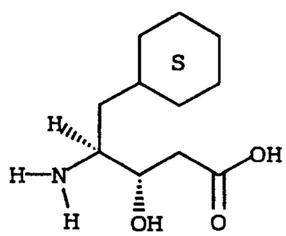

ACHPA

with BOC-ACHPA-OEt being prepared by the method described by Boger et al., J. Med. Chem., 1985, 28, 1779-1790;

4. 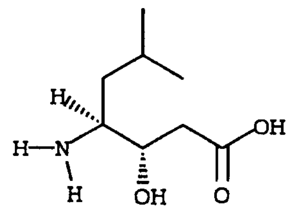

Statine (Sta)

with BOC-Sta-OEt being prepared in accordance with the procedure described by Rich et al., J. Org. Chem., 43, 3624 (1978);

5. 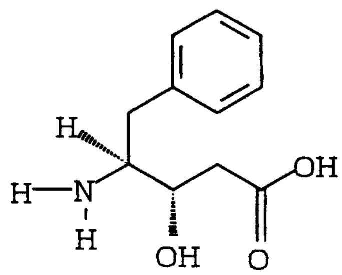

AHPPA

with BOC-AHPPA-OEt being prepared as described by Rich et al., J. Org. Chem., 23, 27-33 (1980).

Other efficient methods for preparing the 2-substituted statine component G, such as 2-isobutyl ACHPA

6. 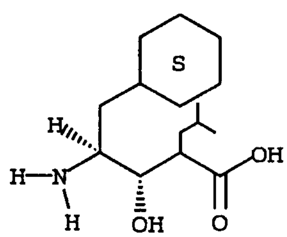

(2-isobutyl)ACHPA

in a suitably protected form are described in Pub. Eur. Pat Appln. 157,409 (with other pertinent references including D. Veber et al, Biochem. Soc. Trans., 12, 956-959 (1984) and H. Stein et al, Fed. Proc. 45, 869, Abstract No 4151 (1986)).

Amide couplings used to form the compounds of this invention are typically performed by the carbodiimide method with reagents such as dicyclohexylcarbodiimide, or N-ethyl, N'-(3-dimethylaminopropyl) carbodiimide. Other methods of forming the amide or peptide bond include, but are not limited to synthetic routes via an acid chloride, aside, mixed anhydride or activated ester. Typically, solution phase amide couplings are performed, but solid-phase synthesis by classical Merrifield techniques may be employed instead.

The selection of protecting groups is, in part, dictated by particular coupling conditions, in part by the peptide analog components involved in the reaction. Such amino-protecting groups ordinarily employed include those which are well known in the art, for example, urethane protecting substituents such as benzyloxycarbonyl (carbobenzoxy), p-methoxycarbobenzoxy, p-nitrocarbobenzoxy, t-butyloxycarbonyl, and the like.

The OH group may be protected by the Bzl (benzyl) group and certain amino groups may be protected by the IPOC group or the 2-chlorobenzyloxycarbonyl (2-C1-CBZ) group. Treatment with HF or catalytic

hydrogenation are typically employed for removal of IPOC or 2-C1-CBZ.

One scheme for preparing compounds of Formula I is presented below. Example 1 specifically illustrates the application of the following Schemes (I and II) to specific compounds.

## SCHEME I

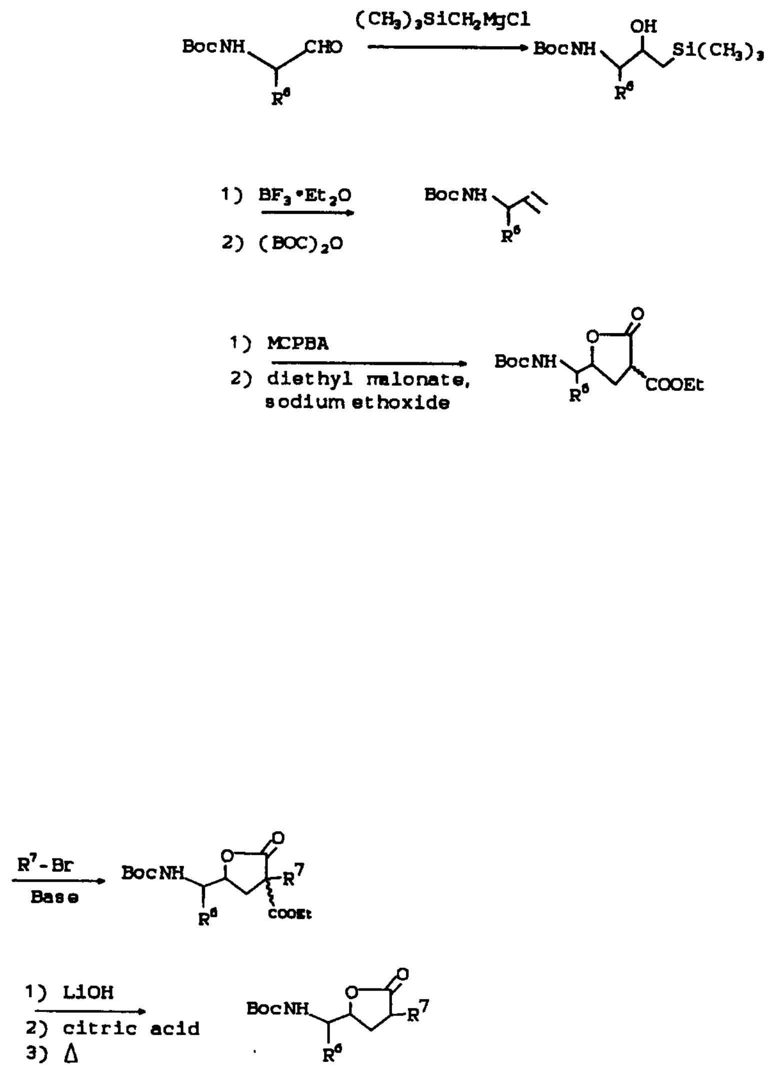

## SCHEME II

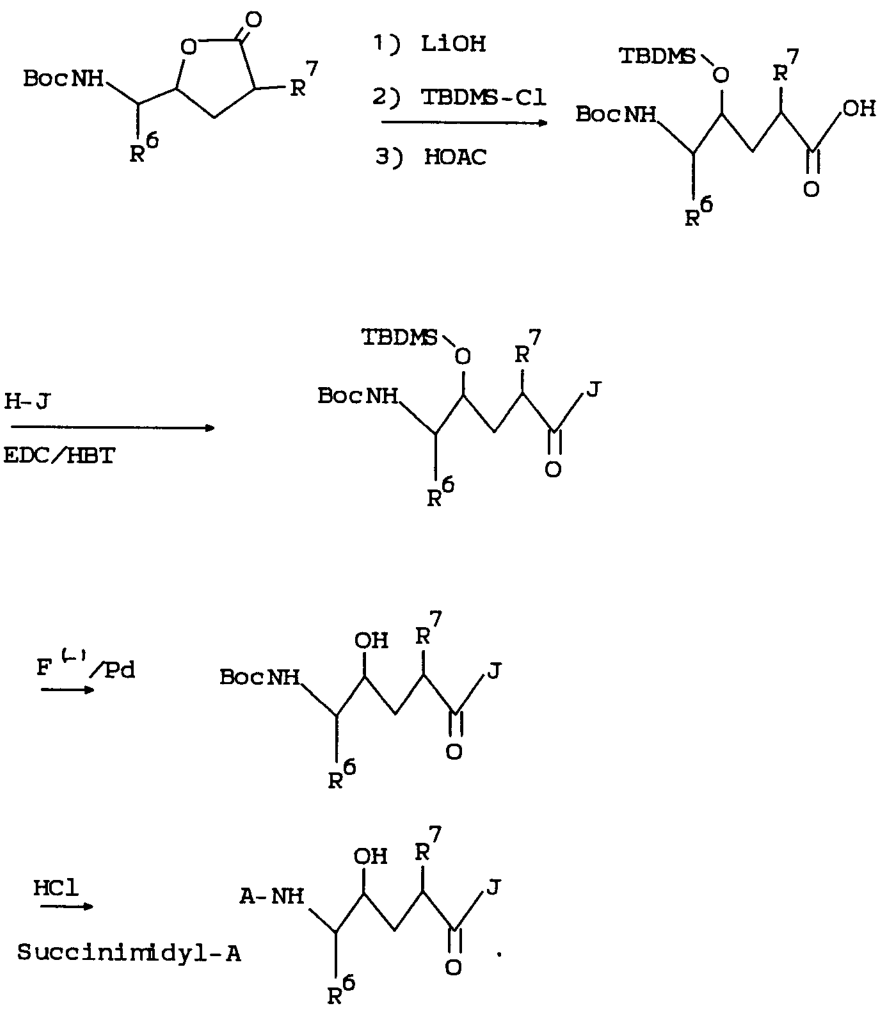


Additional J groups are added by Scheme III.

IIIa: 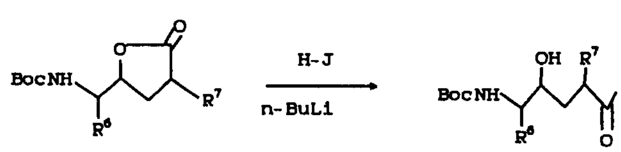


Further derivatization with hydroxyalkyl groups on terminal hydroxyls can be accomplished by reaction with $CsCO_3$, dioxane with the appropriate halogenated reagent in the present of heat. Example 1, Step L illustrates this reaction.

Additional schemes and synthetic methods are formed in EP 0337714, published in October of 1989.

Prodrug esters are synthesized by conventional procedures, including coupling and reduction according to Example 19.

The present invention is also specifically illustrated by the compounds of Tables I-III.

TABLE I

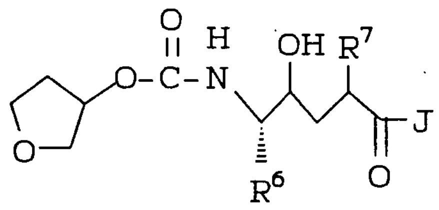

| $R^6$ | $R^7$ | J | mp |
|---|---|---|---|
| $CH_2Ph$ | 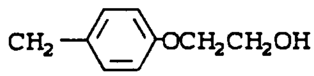 | 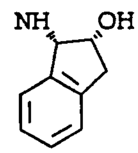 | 127-8°C |
| $CH_2Ph$ | 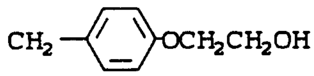 | 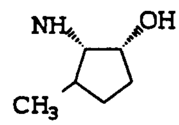 | 202-3°C |
| $CH_2Ph$ | $CH_2Ph$ | 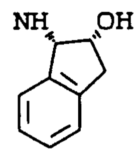 | 225-8°C |
| 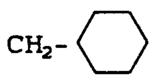 | 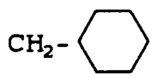 | 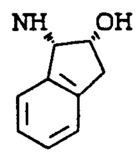 | 187-8°C |
| 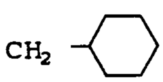 | 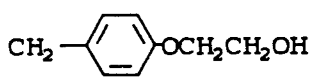 | 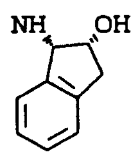 | 201-3°C |
| 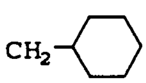 | 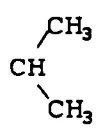 | 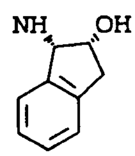 | 199-200°C |
| 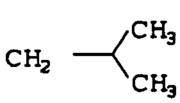 | 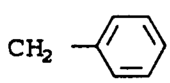 | 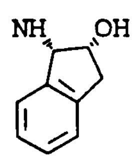 | 210-211. 5°C |

## TABLE II

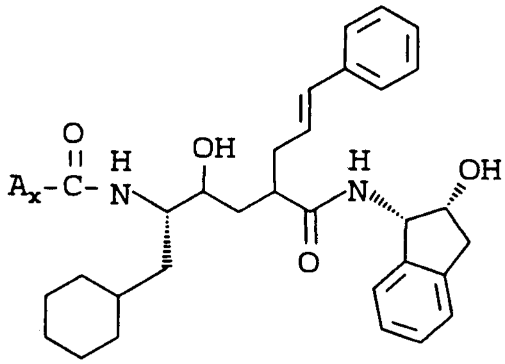

| $A_x$ | mp |
| --- | --- |
| 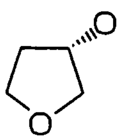 | 159-60°C |
| 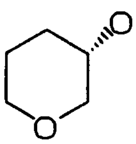 | 169-70°C |
| 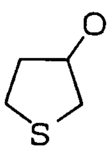 | 178-79°C |
| 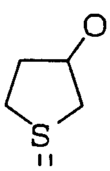 | 115°C |
| 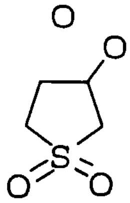 | 110 °C |

| $A_x$ | mp |
|---|---|
| 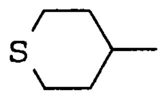 | 180-3°C |
| 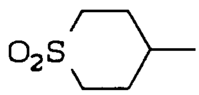 | 141-2°C |
| 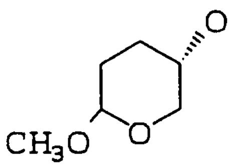 | 152°C |
| 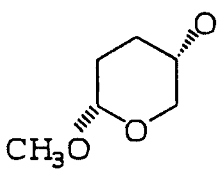 | 180-1°C |
| 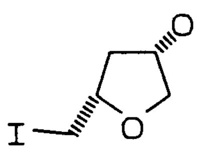 | |

**TABLE III**

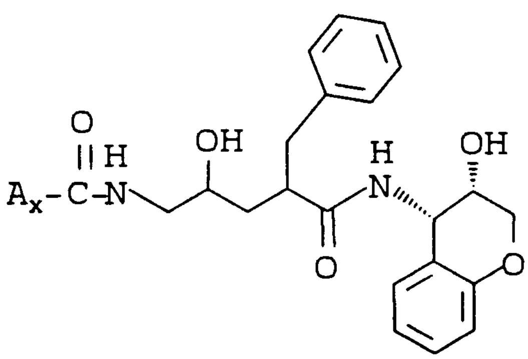

| $A_x$ | mp |
| --- | --- |
| 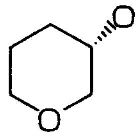 | 165-6°C |
| 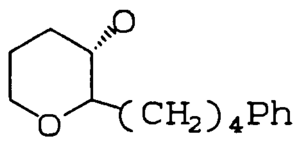 | 189-91°C |
| 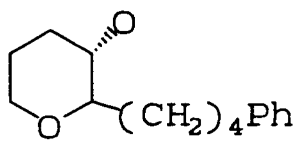 | 183-5°C |
| 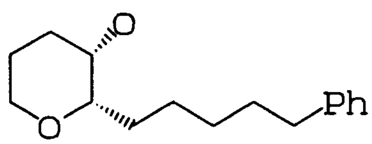 | 184-6°C |
| 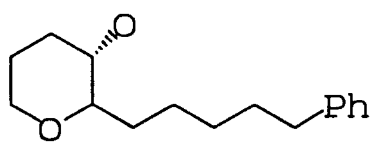 | 173-5°C |

The compounds of the present invention are useful in the inhibition of HIV protease, the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymtomatic, and actual or potential exposure to HIV. For example, the compounds of this invention are useful in treating infection by HIV after suspected past exposure to HIV by e.g., blood transfusion, exchange of body fluids, bite, accidental needle stick, or exposure to patient blood during surgery.

In the present invention, compounds with asymmetric centers may occur as racemates, racemic mixtures and as individual diastereomers, with all isomeric forms of the compounds being included in the present invention.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically-effective amount of a compound of the present invention, or a pharmaceutically-acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting

bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, flourocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV protease-inhibitory compounds with one or more agents useful in the treatment of AIDS.

For example, the compounds of this invention can be given in combination with the antivirals, immunomodulaters, antibiotics or vaccines or other derivative forms thereof as listed in the following Table [source: Marketletter, Nov. 30. 1987, pp. 26-27; Genetic Engineering News, Jan. 1988, Vol. 8, 23]:

TABLE[1]

A. Antivirals

| Drug Name | Manufacturer | Indication |
|---|---|---|
| AL-721 | Ethigen | ARC, PGL |
| BETASERON (interferon beta) | Triton Biosciences | AIDS, ARC, KS |
| CARRISYN (polymannoacetate) | Carrington Labs | ARC |
| CYTOVENE (ganciclovir) | Syntex | CMV |
| DDC (dideoxycytidine) | Hoffmann-La Roche | AIDS, ARC |

---

[1]Abbreviations: AIDS (Acquired Immune Deficiency Syndrome); ARC (AIDS related complex); CMV (Cytomegalovirus, which causes an opportunistic infection resulting in blindness or death in AIDS patients); HIV (Human Immunodeficiency Virus, previously known as LAV, HTLV-III or ARV); KS (Kaposi's sarcoma); PCP (Pneumonocystis carinii pneumonia, an opportunistic infection); PGL (persistent generalized lymphadenopathy).

| Drug Name | Manufacturer | Indication |
|---|---|---|
| FOSCARNET (trisodium phosphonoformate) | Astra AB | HIV inf, CMV retinitis |
| HPA-23 | Rhone-Poulenc Sante | HIV infection |
| ORNIDYL (eflornithine) | Merrell Dow | PCP |
| PEPTIDE T (octapeptide sequence) | Peninsula Labs | AIDS |
| RETICULOSE (nucleophospho-protein) | Advanced Viral Research | AIDS, ARC |
| RETROVIR advanced (zidovudine; AZT) | Burroughs Wellcome | AIDS, ARC pediatric AIDS, KS, asympt HIV, less severe HIV, neurological involvement. |

| Drug Name | Manufacturer | Indication |
|---|---|---|
| RIFABUTIN (ansamycin LM 427) | Adria Labs | ARC |
| (trimetrexate) | Warner-Lambert | PCP |
| UA001 | Ueno Fine Chem Industry | AIDS, ARC |
| VIRAZOLE (ribavirin) | Viratek/ICN | AIDS, ARC, KS |
| WELLFERON (alfa interferon) | Burroughs Wellcome | KS, HIV, in comb with AZT |
| ZOVIRAX (acyclovir) | Burroughs Wellcome | AIDS, ARC, in comb with AZT |

B. Immunomodulators

| Drug Name | Manufacturer | Indication |
|---|---|---|
| ABPP KS (bropirimine) | Upjohn | Advanced AIDS, |
| AMPLIGEN (mismatched RNA) | DuPont HEM Research | ARC, PGL |
| (Anti-human alpha interferon antibody) | Advanced Biotherapy Concepts | AIDS, ARC, KS |

| | | |
|---|---|---|
| Colony Stimulating Factor (GM-CSF) | Sandoz Genetics Institute | AIDS, ARC, HIV, KS |
| CL246,738 (CL246,738) | American Cynamid | AIDS |
| IMREG-1 | Imreg | AIDS, ARC, PGL, KS |
| IMREG-2 | Imreg | AIDS, ARC, PGL, KS |
| IMUTHIOL (diethyl dithio carbamate) | Merieux Institute | AIDS, ARC |

| Drug Name | Manufacturer | Indication |
|---|---|---|
| IL-2 (interleukin-2) | Cetus | AIDS, KS |
| IL-2 (interleukin-2) | Hoffmann-La Roche Immunex | AIDS, KS |
| INTRON-A (interferon alfa) | Schering-Plough | KS |
| ISOPRINOSINE (inosine pranobex) | Newport Pharmaceuticals | ARC, PGL, HIV seropositive patients |
| (methionine enkephalin) | TNI Pharmaceuticals | AIDS, ARC |

| | | |
|---|---|---|
| MTP-PE (muramyl-tripeptide) | Ciba-Geigy | KS |
| THYMOPENTIN (TP-5) (thymic compound) | Ortho Pharmaceuticals | HIV infection |
| ROFERON (interferon alfa) | Hoffmann-La Roche | KS |
| (recombinant erythropoietin) | Ortho Pharmaceuticals | severe anemia assoc with AIDS & AZT therapy |
| TREXAN (naltrexone) | DuPont | AIDS, ARC |
| TNF (tumor necrosis factor) | Genentech | ARC, in combination interferon gamma |

C. Antibiotics

| Drug Name | Manufacturer | Indication |
|---|---|---|
| PENTAM 300 (pentamidine isethionate) | LyphoMed | PCP |

D. Vaccines

Any one of a variety of AIDS or HIV vaccines presently under study and development can be used in combination with the compounds of this invention or salt or derivative forms thereof, in the treatment or prevention of AIDS and diseases of similar character caused by HIV.

It will be understood that the scope of combinations of the compounds of this invention with AIDS antivirals, immunomodulators, antibiotics or vaccines is not limited to the list in the above Table, but includes in principle any combination with any pharmaceutical composition useful for the treatment of AIDS.

SYNTHESIS

The preparation and synthesis follows, in general, U.S. Patent 4,661,473; Evans, B.E. et al, J. Org. Chem., 50, 4615, (1985) and Evans, B.E. et al., "A Stereocontrolled Synthesis of Hydroxyethylene Dipeptide Isosteres," Proc. Am. Pept. Symp., 9, 743-6(1985), and Luly, J.R. et al, J. Org. Chem, 52, 1487 (1987). All temperatures are in degrees centigrade, unless indicated otherwise.

EXAMPLE 1

Preparation of N-(cis-2(R)-hydroxy-1(S)-indanyl)-5(S)-(3(S)-tetrahydrofuranyloxycarbonylamino)-4(S)-hydroxy-6-phenyl-2(R)-[(4-(2-hydroxyethoxy)phenylmethyl]-hexanamide.

Step A: Preparation of N-3(S)-[(1,1-Dimethylethoxycarbonyl)amino]-2(RS)-hydroxy-4-phenyl-1-trimethylsilyl butane:

To a stirred suspension of magnesium turnings (9.79 g, 403 mmol) in dry diethyl ether (200 mL) under nitrogen was added chloromethyltrimethylsilane (50 mL, 358 mmol). The reaction was initiated by gentle warming and then was cooled in an ice bath to maintain gentle reflux. After exotherm was complete the reaction was stirred at room temperature for 1 hour then cooled to -78 °C in a dry ice/acetone bath. To the solution of the Grignard was added dropwise with stirring a solution of N-2(S)-[(1,1-dimethylethoxycarbonyl)-amino]-3-phenyl propionaldehyde (19.3 g, 77.4 mmol) in dry diethyl ether (250 mL) dropwise such that the temperature of the reaction remained below -55 °C. The resultant gray suspension was allowed to warm to room temperature where it was stirred for 30 minutes then was quenched by pouring into a mixture of ice (500 g) and 10% citric acid (500 mL). The organic phase was collected and the aqueous phase was extracted with diethyl ether (3 X 300 mL). The combined organics were washed with 10% citric acid (1 X 300 mL) and brine (1 X 200 mL), dried over anhydrous magnesium sulfate, filtered, and concentrated to give crude N-3(S)-[(1,1-dimethylethoxycarbonyl)amino]-2(RS)-hydroxy-4-phenyl-1-trimethylsilyl butane (26.6 g, quantitative crude yield) as a yellow oil. An analytical sample was obtained by low pressure chromatography (silica gel, 230-400 mesh; diethyl ether: hexanes, 30%:70%) followed by recrystallization from heptane. mp = 91-95 °C;

| elemental analysis. Calcd. for $C_{18}H_{31}NO_3Si$ (337.53): | | | | |
|---|---|---|---|---|
| Found: | C = 64.05,<br>C = 64.05, | H = 9.26,<br>H = 9.13, | N = 4.15;<br>N = 4.22; | [a]$_D$20 = -40.0°. |

Step B: Preparation of 3(S)-Amino-4-phenyl-1-butene.

To a stirred solution of the product of Step A (22.8 g, 67.5 mmoL) in dry methylene chloride (400 mL) cooled in an ice bath and under nitrogen was added in a fine stream boron trifluoride etherate (43 mL, 345 mmol). The solution was allowed to warm to room temperature where it was stirred for 4 days. Reaction was cooled in an ice bath and quenched by the dropwise addition of 10% sodium hydroxide (400 mL). The organic phase was collected and the aqueous phase was extracted with methylene chloride (2 X 250 mL). The combined organics were washed with brine (1 X 200 mL), dried over anhydrous magnesium sulfate, filtered, and concentrated to give crude 3(S)-amino-4-phenyl-1-butene (14.2 g) as a yellow oil.

Step C: Preparation of N-3(S)-[(1,1-Dimethylethoxycarbonyl)amino]-4-phenyl-1-butene:

A solution of the product of Step B (14.2 g) and di-tert-butyl dicarbonate (31.0 g, 142 mmol) in dry methylene chloride (200 mL) was stirred at room temperature for 18 hours, washed with 10% citric acid (3 X 100 mL), water (1 X 100 mL), sat'd. sodium bicarbonate (3 X 125 mL), and brine (1 X 250 mL), dried over anhydrous magnesium sulfate, filtered and concentrated to yield crude N-3(S)-1[(1,1-dimethyl-ethoxycarbonyl)amino]-4-phenylbutene (34.6 g) as a yellow oil. Crude product was purified by low pressure chromatography (silica gel, 230-400 mesh, 10 X 20 cm column; diethylether: hexanes, 20%: 80%) to yield N-3(S)-[(1,1-dimethylethoxylcarbonyl)amino]-4-phenyl-1-butene (16.3 g, 97.6% yield) as a white solid. An analytical sample was obtained by recrystallization from heptane. mp = 67.5-68.5°C;

| elemental analysis, Calcd. for $C_{15}H_{21}NO_2$ (247.34): | | | |
|---|---|---|---|
| Found: | C = 72.84,<br>C = 72.78, | H = 8.56,<br>H = 8.76, | N = 5.66.<br>N = 5.64. |

Step D: Preparation of 1(R)-[1'(S)-(1,1-Dimethylethoxycarbonyl)amino-2-phenylethyl]oxirane:

To a solution of the product of Step C (9.4 g, 38 mmol) in dry methylene chloride (100 mL) cooled in an ice bath and under nitrogen was added 3-chloroperoxybenzoic acid (technical grade, 80-85%; 41 g, 200 mmol). The mixture was stirred at 0°C for 18 hours and 25°C for 23 hours, then diluted with diethyl ether (300 mL), and poured in ice cold aqueous 10% sodium sulfite (1 L). The organic layer was collected and the aqueous layer was extracted with diethyl ether (3 X 100 mL). The combined organics were washed with 10% sodium sulfite (3 X 100 mL), satd. sodium bicarbonate (3 X 100 mL), and brine (1 X 100 mL), dried over anhydrous sodium sulfate, filtered and concentrated to give a white solid. Crude product was purified by low pressure chromatography (silica gel 230 - 400 mesh, 8 X 15 cm column; ethyl acetate: hexanes, 25%:75%) to yield 1(R)-[1'(S)-(1,1-dimethylethoxycarbonyl)amino-2-phenylethyl]oxirane(7.0 g, 70% yield) as a clear oil which crystallized upon standing. An analytical sample was obtained by recrystallization from heptane. mp = 51.5-52°C;

| elemental analysis, Calcd. for $C_{15}H_{21}NO_2$ (263.34): | | | | |
|---|---|---|---|---|
| Found: | C = 68.42,<br>C = 68.22, | H = 8.04,<br>H = 8.26, | N = 5.32.<br>N = 5.29; | [a]$_D$20 = 1.34°. |

Step E: Preparation of(5S,1'S)-3-carboethoxy-5-(1-((1',1'-dimethylethoxycarbonyl)amino)-2-phenylethyl)-dihydrofuran-2-(3H)-one.

The product from Step D, 9.93 g, was dissolved in 100 mL of absolute ethanol and added to a solution of 2.6 g of sodium and 20.1 mL of diethyl malonate in 170 mL of absolute ethanol. After stirring overnite, the reaction was acidified to pH 4 with 10% citric acid and extracted with 2 X 500 mL of ether. The combined organic extracts were washed 1 X 500 mL $H_2O$, 1 X 500 mL sat'd $NaHCO_3$, 1 X 500 mL sat'd brine and dried over $MgSO_4$. The solvents were removed and the crude product purified by low pressure chromatography on silica gel eluting with 50% ether/hexanes (or EtOAc/hexanes). The yield of semi-solid product was 10.6 g. The later fractions contained 2.5 g of the undesired 5 R isomer as a white solid.

Step F: Preparation of (5S,1'S)-3-carboethoxy-3-(4-benzyloxyphenylmethyl)-5-[1-(1,1-dimethylethoxycarbonyl)amino)-2-phenylethyl]dihydrofuran-2-(3H)-one

To a stirred solution of (5S,1'S)-3-carboethoxy-5-[1-((1',1'-dimethylethoxycarbonyl)amino)-7-phenylethyl)-dihydrofuran-2-(3H)-one (product of Step E), 2 g (5.3 mmol) in 25 mL of absolute ethanol was added a solution of 0.13 g of sodium in 2.2 mL of absolute ethanol followed by 1.30 g (5.5 mmol) of 4-benzyloxybenzyl chloride. The solution was heated to 50°C under nitrogen for 1 hour, then cooled in an ice bath and acidified with 20 mL of 10% citric acid and diluted with 200 mL of water. The mixture was extracted with 3 X 100 mL of ether and the combined ether extracts washed with 50 mL of water, 200 mL of sat'd $NaHCO_3$ and dried over $MgSO_4$. Removal of solvents under reduced pressure and purification by low pressure chromatography on silica gel, eluting with 40% ether in hexanes gave 1.56 g (51% yield) of a clear colorless glass essentially homogeneous by TLC (50% ether/hexanes).

Step G: Preparation of (3R,5S,1'S)-3-(4-benzyloxyphenylmethyl)-5-(1((1,1-dimethylethoxycarbonyl)amino)-2-phenylethyl)-dihydrofuran-2-(3H)-one.

The product of Step F, 13.6 g, was dissolved in 250 mL of 1,2-dimethoxyethane, and to it was added 117 mL of 1 M lithium hydroxide at room temperature. After stirring for 12 hours, the solvents were removed under reduced pressure, the residue suspended in 200 mL of 10% citric acid and extracted 3 X 500 mL of diethyl ether. The combined ether extracts were washed with 500 mL of brine, dried ($MgSO_4$) and the concentrated to dryness. The residue was dissolved in 250 mL of toluene, heated to reflux for 12 hours, then concentrated to dryness under reduced pressure. Purification by medium pressure chromatography over silica gel eluting with 15% ethyl acetate/hexanes gave 3.2 g of the 3R-lactone as a clear foam. Further elution with the same solvents gave 6.15 g of the 3S-lactone as a white solid.

Step H: Preparation of N'-(1,1-dimethylethoxycarbonyl)-5(S)-amino-4(S)-(1',1'-dimethylethyl-1,1-dimethylsilyloxy)-6-phenyl-2(R)-(4-benzyloxyphenylmethyl-hexanoic acid.

The product of Step G, 0.6 g, was dissolved in 30 mL of a 2:1 mixture of ethylene glycol dimethyl ether/water, and to it was added 5 mL of 1 M lithium hydroxide at room temperature. After stirring for 1 hour, the mixture was partitioned between 200 mL chloroform and 20 mL 10% citric acid. The layers were separated and the aqueous phase extracted with 3 X 20 mL chloroform. The combined organic layers were dried ($Na_2SO_4$) and the solvent removed to yield 0.56 g of the crude hydroxy acid. This residue was dissolved in 5 mL of dry DMF and 0.845 g tert-butyl dimethylsilyl chloride and 0.725 g of imidazole were added. After stirring for 18 hours, the reaction was poured into 50 mL of water and extracted with 3 X 20 mL of ethyl acetate. The combined organic extracts were washed with 3 X 20 mL of 10%. citric acid, 1 X 20 mL of water, 3 X 10 mL of saturated aqueous solution of $Na_2CO_3$, and 20 mL of brine. After drying ($Na_2SO_4$), the solvent was removed and the resulting residue dissolved in a mixture of 5 mL of THF, 5 mL of glacial acetic acid, and 2 mL of water. The mixture was stirred for 4 hours, then poured into 50 mL of water and extracted with 3 X 20 mL of ether. The combined ether extracts were washed with 2 X 20 mL of water, brine, dried ($Na_2SO_4$), and the solvent removed. Purification by medium pressure chromatography over silica gel, eluting with MeOH/$CHCl_3$ gave 0.60 g of the product as a white glassy solid.

Step I: Resolution of 1-Amino-2-hydroxyindan

From the known racemic 1-amino-2-hydroxyindan, the resolution was carried out as described for the 3-amino-1,2-dihydroxyindan in Example 7 below (Steps D and E). The (1S,2R)-1-amino-2-hydroxyindan

resulting from saponification of the higher $R_f$ diastereomer was shown to have an $a_D$ of -58° (c = 1.0, $CHCl_3$). The (1R, 2S)-1-amino-2-hydroxyindan resulting from saponification of the lower $R_f$ diastereomer was found to have an $a_D$ of +62° (c = 1.0, $CHCl_3$).

Stem J: Preparation of N-(2(R)-hydroxy-1(S)-indanyl)-5(S)-(1,1-dimethylethoxycarbonylamino)-4(S)-hydroxy-6-phenyl-2(R)-(4-benzyloxyphenylmethyl) hexanamide

The product from Step H, 0.12 g, was dissolved in 2 ml dry DMF and to it was added 40 mg of 1(S)-amino-2(R)-hydroxyindane, (Step I) 25 mg of 1-hydroxybenzotriazole hydrate and 70 mg of dimethyl-3-(3-dimethyl aminopropyl)carbodiimide hydrochloride. Triethylamine was added to the stirred solution until the pH was 8.5 (32 mL). After stirring for concentrated to dryness under reduced pressure, the residue was dissolved in 100 mL of chloroform and worked with 1 X 50 mL of 10% citric acid, 1 X 50 mL $H_2O$, 1 X 50 mL sat'd $NaHCO_3$, dried over $MgSO_4$ and concentrated to dryness. The residue was dissolved in 1 mL of tetrahydrofuran and added to 2 mL of 1 M tetrabutylammonium fluoride in THF. After stirring overnight at room temperature the reaction mixture was diluted with 10 mL of 10%. citric acid and the white precipitate collected by filtration. The product was purified by low pressure chromatography on silica gel eluting with 2% methanol/$CH_2Cl_2$ to give 85 mg of product which was essentially homogeneous by TLC (3% methanol/$CH_2Cl_2$).

Step K: Preparation of N-(2(R)-hydroxy-1(S)-indanyl)-5(S)-(1,1-dimethylethoxycarbonylamino)-4(S)-hydroxy-6-phenyl-2(R)-(4-hydroxyphenylmethyl)hexanamide

The product of Step J, 85 mg was dissolved in 10 mL of methanol and 10 mL of THF, and to it was added 0.10 g of 10% palladium on carbon. The mixture was stirred under an atmosphere of hydrogen for 48 hours at room temperature, then filtered and concentrated to dryness. The residue was dissolved in 10 mL of hot ethanol and 20 mL water was added. On cooling the white solid precipitate was collected and dried under vacuum over $P_2O_5$. The yield was 72 mg (98% yield) of pure product: mp 218-219°C (effervesces, sinters at 215) elemental analysis,

| Calc'd for $C_{33}H_{40}N_2O_6$: (560.696): | | | |
|---|---|---|---|
| | C, 70.69; | H, 7.19; | N, 5.00; |
| Found: | C, 70.62; | H, 7.39; | N, 4.79. |

Step L: Preparation of N-(cis-2(R)-hydroxy-1(S)-indanyl)-5(S)-[1,1-dimethylethoxycarbonylamino)-4(S)-hydroxy-6-phenyl-2(R)-[(4-(2-hydroxyethoxy)phenyl]methyl]-hexanamide

A stirred mixture of Step K product, N-(2(R)-hydroxy-1(S)-indanyl)-5(S)-[1,1-dimethylethoxy carbonyl)-amino]-4(S)-hydroxy-6-phenyl-2(R)-(4-hydroxyphenylmethyl) hexanamide (1.0 g, 1.8 mmol), and anhydrous cesium carbonate (2.0 g, 6 mmol) in 140 mL of anhydrous dioxane was heated to 80°C (internal temperature) for 3 hrs. To this mixture was added 9 mL of ethyl bromoacetate. The mixture was kept at this temperature for 20 hours. After cooling to room temperature the mixture was diluted with chloroform (100 mL), filtered, concentrated to dryness under reduced pressure.

The resulting ester was dissolved in 200 ml of dry 1,2-dimethoxyethane and 5 ml of 2M lithium borohydride in tetrahydrofuran was added. After stirring for 2 hours, the reaction was quenched with 50 mL of 5% citric acid, and extracted with 200 mL of chloroform.

The organic extract was washed with 100 mL of water, 100 mL of saturated $NaHCO_3$ and dried over $MgSO_4$. Concentration to dryness under reduced pressure gave 1.2 g of product as white solid.

| Elemental analysis: calcd for $C_{35}H_{44}N_2O_7 \cdot 0.25H_2O$ (609.252) | | | |
|---|---|---|---|
| | C, 69.00; | H, 7.36; | N, 4.59 |
| Found: | C, 68.82; | H, 7.35; | N, 4.21 |

Step M: Preparation of 3(S)-tetrahydrofuranyl succinimidyl carbonate:

A solution of 20 mL of 12.5% phosgene in toluene and 1.0 g of (S)-(+)-3-hydroxytetrahydrofuran was aged in a stoppered flask for 48 hours. The solvents were removed under reduced pressure and the residue dissolved in 30 mL of anhydrous acetonitrile, then cooled in an ice bath. To this cold solution was added 1.7 g of N-hydroxysuccinimide and 1.9 mL of triethylamine. The mixture was aged for 12 hours at 25°C, then concentrated to dryness. The residue was dissolved in 200 mL of ethyl acetate, washed with 2 x 50 mL of water, dried over $MgSO_4$ and concentrated to dryness under reduced pressure. The oily residue was dissolved in 10 mL of ethyl acetate passed through a 300 mL of silica gel, eluting with ethyl acetate. Concentration of the eluate to dryness gave 2 g of product as a white crystalline solid.

Step N: Preparation of N-(2(R)-hydroxy-1(S)-indanyl)-5(S)-(3(S)-tetrahydrofuranyloxycarbonylamino)-4(S)-hydroxy-6-phenyl-2(R)-[4-(2-hydroxyethoxy)phenylmethyl]hexanamide

To a suspension of 0.200 g of the product of Step L, in 20 mL of dichloromethane was added 2 mL of 1.4M HCl in ether. After stirring overnight at 25°C, the mixture was concentrated to dryness and diluted with 30 mL of $CH_2Cl_2$. To this mixture was added 80 mg of the product of Step M, 3(S)-tetrahydrofuranyl succinimidyl carbonate, and 0.06 mL of triethylamine. After stirring overnight at 25°C, the mixture was concentrated to dryness, and the product purified by preparative thin-layer chromatography (10% methanol in $CHCl_3$). The product, 0.12 g, was obtained as a white crystalline solid after drying: mp 127-8°C,

| elemental analysis. Calcd for $C_{35}H_{42}N_2O_8 \cdot 0.25H_2O$ (623.24): | | | |
|---|---|---|---|
| | C, 67.45; | H, 6.87; | N, 4.49. |
| Found: | C, 67.32; | H, 6.83; | N, 4.44. |

EXAMPLE 2

Preparation of N-(2R)-hydroxy-1(S)-indanyl)-5(S)-(3(S)-tetrahydrofuranloxycarbonylamino)-4(S)-hydroxy-2(R)-(4-(2-hydroxyethoxy)phenyl)methyl-6-cyclohexyl-hexanamide

Step A: Preparation of (5S,1'S)-5-(1'-((1,1-dimethylethoxy-carbonyl)amino)-2'-cyclohexylethyl)-4,5-dihydrofuran-2-(3H)-one:

A solution of the (5S,1'S)-5-(1'-((1,1-dimethylethoxy-carbonyl)amino)-2'-phenylethyl)-4,5-dihydrofuran-2-(3H)-one was dissolved in ethyl acetate and rhodium on alumina was added. This mixture was shaken under a hydrogen atmosphere (50 psi) at 50°C overnight. Filtration and evaporation of the solvent afforded the title compound as a viscous oil, which solidified as a hard glass.

Step B: Preparation of 4-Benzyloxybenzyl iodide:

A mixture of 25 g of commercial 4-benzyloxybenzyl chloride, 32 g of NaI and 107 mL of acetone was stirred in the dark for 5 hours. The mixture was diluted with 100 mL of acetone, filtered, and concentrated under reduced pressure to an orange solid. The solid was dissolved in 400 mL of ether, washed with 75 mL of water, 2 x 40 mL of 10% $NaHSO_3$, 75 mL of water and dried ($MgSO_4$). The solution was concentrated to 125 mL and 200 mL of hexane added with stirring and cooling (0°C). The product was collected by filtration and dried under vacuum.

Step C: Preparation of (3R,5S,1'S)-3-(4-benzyloxyphenylmethyl)-5-(1((1,1-dimethylethoxycarbonyl)amino)-2'-cyclohexylethyl)4,5-dihydrofuran-2-(3H)-one:

To a solution of 23 mL of diisopropylamine in 100 mL of tetrahydrofuran (THF) at 0°C was added 0.164 moles of n-butyllithium. After 15 minutes at 0-4°C the solution was cooled to -78°C and a solution of 25 g of the product of Step A in 125 mL of THF was added over 1 hour, keeping the temperature below -72°C. The reaction was aged at -54°C for 1 hour, then recooled to -78°C and a solution of 26 g of 4-benzyloxybenzyl iodide in 110 mL of THF was added over 80 minutes keeping the temperature below -74°C. The reaction aged at -78°C for 2.5 hours, then at -54°C for 1.5 hours and quenched at -50°C with

250 mL of 1M $NaHSO_4$. The mixture was extracted with 3 x 160 mL of ethyl acetate and the extracts washed with water, 10% $NaHSO_3$. satd. NaCl and dried ($MgSO_4$). Trituration with 15% ethyl acetate in hexanes gave 13.7 g of product as a tan solid. Chromatography of the mother liquors, eluting with 15% ethyl acetate hexanes gave an additional 8.1 g of product.

Step D: Preparation of N'-(1,1-dimethylethoxycarbonyl)-5(S)-amino-4(S)-(1',1'-dimethylethyl-1,1-dimethylsilyloxy)-6-cyclohexyl-2(R)-(-4-benzyloxyphenylmethyl)hexanoic acid:

The product of Step C, 11.8 g, was dissolved in 350 mL of dimethoxyethane and 350 mL of 0.5 N LiOH. After stirring 4 hours, the mixture was concentrated to 300 mL and acidified with 1L of 5% citric acid. The white solid product was collected by filtration and dried at 60°C under vacuum for 12 h. The hydroxy-acid (11.6 g) was dissolved in a mixture of DMF (150 mL), imidazole (58.7 g) and tertbutyl- dimethylchlorosilane (58.7 g). After stirring at 25°C for 48 hours, 170 mL of methanol was added. The mixture was aged for 30 minutes at 25°C, then concentrated under reduced pressure to near dryness. The residue was diluted with 250 mL of 10% citric acid, then extracted with 4x600 mL of ethyl acetate. The extracts were washed with water, dried over $MgSO_4$ and concentrated to dryness. The product (14.9 g) was obtained as a clear resin.

Step E. Preparation of N-((2(R)-hydroxy-1(S)-indanyl)-5(S)-(1,1- dimethylethoxycarbonyl)-4(S)-(1',1'-dimethylethyl-1,1-dimethylsilyloxy)-6-cyclohexyl-2(R)-(4-benzyloxyphenylmethyl)hexanamide.

The product of step E, 14.9 g, 1-hydroxybenzotriazole hydrate, 3.26 g, dimethyl-3-(3-dimethylaminopropyl)carbodiimide, 7.04 g, 4-methylmorpholine, 2.8 mL, and 250 mL of DMF were stirred at 25°C for 12 hours. The mixture was concentrated to dryness under reduced pressure, dissolved in 300 mL of ethyl acetate, washed with 150 mL of 10% citric acid, water, sat'd $NaHCO_3$ and dried ($MgSO_4$). The residue was purified by chromatography on silica gel. Elution with 30% ethyl acetate in hexanes gave 10.1 g of a foamy resin.

Step F. Preparation of N-((2(R)-hydroxy-1(S)-indanyl-5(S)-(1,1-dimethylethoxycarbonyl)-4(S)-(1',1'-dimethylethyl-1,1-dimethylsilyloxy)-6-cyclohexyl-2(R)-(4-hydroxyphenylmethyl)hexanamide.

The product of step E, 10.1 g, and 6 g of 10% palladium on carbon in 600 mL of ethyl acetate was stirred under an atmosphere of hydrogen for 12 h, filtered through celite and concentrated to dryness under reduced pressure. The product, 9.5 g, was obtained as a glass.

Step G:

To a stirred solution of the product Step F (800 mg) in 20 mL of dioxane was added cesium carbonate (1.15 g) and the mixture heated to 80°C under argon. Ethyl bromoacetate (0.195 mL) was added in one portion, and heating continued for 3.5 hours. After cooling to ambient temperature, the mixture was diluted with 100 mL of chloroform, filterd, and the solids washed with three 30 mL portions of chloroform. Removal of the solvents under reduced pressure gave 918 mg of the ester as a solid. To a stirred solution of the ester in 16 mL of DME was added 2.3 mL of a 2.0 M solution of lithium borohydride in THF slowly. After stirring under argon for 1.5 hours, the mixture was carefully quenched with 20 mL of 1M aqueous citric acid, concentrated under reduced pressure, and extracted with two 40 mL portions of ethyl acetate. The combined organic layers were washed with water, 10% sodium carbonate, brine and dried over magnesium sulfate. Removal of solvents under reduced pressure gave 853 (100%) of the silyl protected hydroxyethyl compound as a colorless foam.

Step H:

The product from Step G (2.55 g) was dissolved in 32 mL of 1M tetrabutylammonium fluoride in THF and stirred under argon atmosphere for 48 hours, then warmed to 35°C for 4 hours. After cooling, the mixture was poured into a stirred solution of 45 mL of 1M citric acid and 150 mL of ice-water. After stirring for 15 minutes, the precipitate was collected by filtration and washed with water. Recrystallization from 1:1 EtOAc-hexane gave 1.51 g of colorless crystals: mp 167-168°C,

| | | | |
|---|---|---|---|
| anal: Calc'd for $C_{35}H_{50}N_{2}O_{7}$ | C 68.83 | H 8.25 | N 4.59 |
| Found | C 68.44 | H 8.30 | N 4.61 |

Step I:

The product from Step H (65 mg) was dissolved in 4 mL of $CH_2Cl_2$ and treated at 0°C with 2 mL of trifluoroacetic acid. After stirring for 1 hour in the cold, the solvents were removed at reduced pressure. The residue was dissolved in 25 mL of $CHCl_3$, washed with 10 mL of saturated $NaHCO_3$, dried over $Na_2SO_4$ and concentrated under reduced pressure. To a stirred solution of the oily residue in 2 mL of $CH_2Cl_2$ was added 37 mg (0.16 mmol) of 3(S)-tetrahydrofuranyl succinimidyl carbonate and 0.077 mL of triethylamine. After stirring overnight at 25°C, the mixture was diluted with 20 mL of $CHCl_3$ and washed with 10 mL of water and brine, then dried over $Na_2SO_4$. Removal of solvents under reduced pressure gave a solid. Crystallization from ethyl acetate containing a trace of ethanol gave 39 mg (59%) of the title compound as a colorless solid: mp 201-203°C.

| | | | |
|---|---|---|---|
| Anal. Calc'd for $C_{35}H_{48}N_{2}O_{8}$ | C 67.29 | H 7.74 | N 4.48 |
| Found | C 66.99 | H.7.71 | N 4.27 |

EXAMPLE 3

Preparation of N-(2(R)-hydroxy-1(S)-indanyl)-5(S)-(3(S)-tetrahydrofuranyloxycarbonylamino)-4(S)-hydroxy-6-phenyl-2(R)-phenylmethyl hexanamide

Step A: Preparation of (1S,2R)-3-(3-phenylpropionyl)-2,2-dimethyl-[3,3a,4,8b]tetrahydro-2H-indeno-[2.1-d]-oxazole

A mixture of 1.7 g of 3-phenylpropionic acid, 1.5 g of (1S,2R)-1-amino-2-hydroxy indane, 1.4 g of 1-hydroxybenzotriazole hydrate, 2.8 g of dimethyl-3-(3-dimethylaminopropyl)carbodiimide hydrochloride, 2.1 mL of triethylamine and 20 mL of dry DMF was stirred overnight. The mixture was concentrated to dryness under reduced pressure, the residue dissolved in 200 mL of chloroform and washed with 100 mL 5% citric acid, 100 mL water and 100 mL saturated sodium bicarbonate. The residue after drying was dissolved in 50 mL of methylene chloride, 20 mL 2,2-dimethoxypropane and 10 mL of acetone with 200 mg p-toluenesulfonic acid monohydrate.

After stirring for 2 days at room temperature, 50 mL of saturated sodium bicaronate was added and the organic layer separated, dried over $MgSO_4$ and concentrated to dryness under reduced pressure. The residue, 3.21 g, crystallized on standing. The product was homogeneous by TLC.

Step B: Preparation of N-(2(R)-hydroxy-1(S)-indanyl)-5(S)-(1,1-dimethylethoxycarbonylamino)-4(S)-hydroxy-6-phenyl-2(R)-phenylmethyl hexanamide

A solution of the product of Step A, 2.16 g, and 1(R)-[1'(S)-1,1-dimethylethoxycarbonyl)amino-2-phenylethyl]oxirane (the product of Step D, Example 1), 1.77 g, in 45 mL of dry THF was cooled to -78°C under nitrogen atmosphere. To this cold, stirred solution was added 13.5 mmole of commercial n-butyllithium in hexane over 10 minutes. After aging the solution 1 hour at -78°C, 1 hour at -40°C, then 4 hours at -25°C, the reaction was quenched with 50 mL of water and extracted with 2 x 10 mL of ethyl acetate. The combined organic extracts were dried and concentrated to a resin.

Trituration with ether (ca. 10 mL) and hexanes (ca. 50 mL) in an ice bath gave, after aging overnight, 1.38 g of product. Further drying under vacuum gave 1.32 g of a solid. This solid was dissolved in 16 mL of methanol and cooled in an ice bath. To this cold solution was added 120 mg of camphorsulfonic acid and 0.230 mL of ethylene glycol. The solution was aged at room temperature for 12 hours, then cooled in ice bath, diluted with ether (10 mL) and filtered. The solid product weighed 1.2 g after drying.

Step C: Preparation of N-(2(R)-hydroxy-1(S)-indanyl)-5(S)-(3(S)-tetrahydrofuranyloxycarbonylamino)-4(S)-hydroxy-6-phenyl-2(R)-phenylmethyl hexanamide

The product of Step B, 1.2 g, was suspended in 20 mL of dichloromethane and cooled in an ice bath to 0 ° C. To this stirred solution was added 10 mL of anhydrous trifluoroacetic acid. After 0.5 hours, the solution was concentrated to dryness under reduced pressure. The residue was dissolved in 100 mL of dichloromethane and washed with 100 mL 0.1N sodium hydroxide, dried over $MgSO_4$ and filtered. To this solution was added 0.6 g of 3(S)-tetrahydrofuranyl succinimidyl carbonate and 0.3 mL of triethylamine. After stirring overnight, the mixture was concentrated to dryness and the product purified by low pressure chromatography using 5% methanol in chloroform. There was obtained after drying 0.92 g of product as a white crystalline solid: mp 225-8 ° C,

| Elemental Analysis calcd for $C_{33}H_{38}N_2O_6 \bullet 0.2CHCl_3$ (582.557): | | | |
|---|---|---|---|
| | C, 68.45; | H, 6.61; | N, 4.81. |
| Found: | C, 68.17; | H, 6.62; | N, 4.69. |

EXAMPLE 4

Preparation of N-(2(R)-hydroxy-1(S)-indanyl)-5(S)-(3(S)-tetrahydrofuranyloxycarbonylamino)-4(S)-hydroxy-6-cyclohexyl-2(R)-(*trans*-3-phenyl-prop-2-en-1-yl) hexanamide

Employing the procedure substantially as described in Example 2, Steps A through I, but substituting cinnamyl bromide for the 4-benzyloxybenzyl iodide in Step C, there was obtained the product as a white crystalline solid: mp 159-60 ° C.

EXAMPLE 5

Preparation of N-(2(R)-hydroxy-1(S)-indanyl)-5(S)-(3(S,R)-tetrahydrothiofuranyloxycarbonylamino)-4(S)-hydroxy-6-cyclohexyl-2(R)-(3-phenyl-2-propen-1-yl)-hexanamide

Employing the procedure substantially as described in Example 4, but substituting 3-tetrahydrothiofuranyl succinimidyl carbonate (from 3-hydroxytetrahydrothiofuran in the manner described in Example 1, Step M) for the 3(S)-tetrahydrofuranyl succinimidyl carbonate there was obtained a white solid: mp 178-9 ° C.

EXAMPLE 6

Preparation of N-(2(R)-hydroxy-1(S)-indanyl)-5(S)-(1-oxo-3(S,R)-tetrahydrothiofuranyloxycarbonylamino)-4-(S)-hydroxy-6-cyclohexyl-2(R)-(3-phenyl-2-propen-1-yl)-hexanamide

N-(2(R)-hydroxy-1(S)-indanyl)-5(S)-(3(S,R)-tetrahydrothiofuranyloxycarbonylamino)-4(S)-hydroxy-6-cyclohexyl-2(R)-(3-phenyl-2-propen-1-yl)-hexanamide, 0.018 g, was dissolved in 3 mL of methanol and cooled to 0°C. To this solution was added 0.031 g of $NaIO_4$ followed by 1 mL of water. After stirring for 4 hours at 0°C., the solvents were evaporated and the resulting residue was taken up in ethyl acetate and washed with water and dried over anhydrous $Na_2SO_4$. Filtration and evaporation of the solvent gave a residue which was chromatographed over silica gel (5% methanol/chloro-form) to afford the title compound as a white solid (mp 115°C.)

EXAMPLE 7

Preparation of N-[cis-2(R)-hydroxy-1(S)-indanyl]-5(S)-(1,1-dioxo-3(S,R)-tetrahydrothiofuranyloxycarbonylamino)-4(S)-hydroxy-6-cyclohexyl-2(R)-(trans-3-phenyl-2-propen-1-yl)-hexanamide

N-(2(R)-hydroxy-1(S)-indanyl)-5(S)-(3(S,R)-tetrahydrothiofuranyloxycarbonylamino)-4(S)-hydroxy-6-cyclohexyl-2(R)-(3-phenyl-2-propen-1-yl)-hexanamide, 0.013 g, was dissolved in 4 mL of methanol. The solution was cooled to 0°C. and 0.039 g of oxone was added followed by 3 mL of water. After stirring for 12 hours, the solvents were evaporated and the resulting residue was taken up in ethyl acetate and washed with water, saturated aqueous $NaHCO_3$, and dried over anhydrous $Na_2SO_4$. Filtration and evaporation of the solvent gave a residue which was chromatographed over silica gel (5% methanol/chloroform) to afford the title compound as a white solid (mp 110°C.)

The following alcohols were prepared as described below and incorporated into the compounds shown in the Tables, using methods substantially as described in Examples 1-7.

EXAMPLE 8

Preparation of 4-Hydroxytetrahydrothiopyran

Tetrahydrothiopyran-4-one, 2.0 g, was dissolved in 20 mL of absolute ethanol and added to a stirred suspension of $NaBH_4$ 1.3 g in 20 mL of absolute ethanol at 0°C. After stirring for 15 minutes, the resulting mixture was acidified with 10% aqueous citric acid to pH 6. Evaporation of the solvents provided a residue which was extracted with ethyl acetate (2x20 mL). Evaporation of the solvent yields the title compound as a white solid.

EXAMPLE 9

Preparation of 3(R,S)-Hydroxytetrahydrothiofuran

To a stirred solution of tetrahydrothiofuran-3-one, 2.0 g, in 15 mL of absolute ethanol at 0°C was added of 0.89 g of $NaBH_4$ in 10 mL of absolute ethanol dropwise for 5 min. The resulting mixture was stirred at 0°C for 30 min and then it was acidified with 10% aqueous citric acid to pH 6. Evaporation of the solvents provides a residue which was extracted with ethyl acetate (2x20 mL). The combined extracts were dried over anhydrous $Na_2SO_4$ and evaporated to yield 1.4 g of the title compound as a white solid.

EXAMPLE 10

Preparation of 6(R)-methoxy-3(S)-hydroxytetrahydro-2H-pyran and 6(S)-methoxy-3(S)-hydroxy-tetrahydro-2H-pyran

To a stirred suspension of (-)-diisopinocamphenylborane (100 mmol in 40 ml of THF) at 0°C was added 9.1 ml of 6(SR)-methoxy-2,3-dihydro-2H-pyran. The reaction mixture was stirred at 0°C for 12h. After this period, the reaction temperature was raised to 25°C and 22.4 ml of freshly distilled acetaldehyde was added dropwise for 30 min. The resulting mixture was stirred at 25°C for 6h and then excess of acetaldehyde was removed under reduced pressure and to it 80 ml of THF was added. The boronate thus obtained was oxidized with 100 ml of 3 N sodium hydroxide and 12.5 ml of 30% hydrogen peroxide. The reaction mixture was stirred at room temperature for 6h and then diluted with 200 ml of ether. The layers were separated and the aqueous layer was extracted with ether (2x100 ml). The combined organic extracts were dried over anhydrous $Na_2SO_4$ and evaporated. The residue was loaded over a silica gel column and eluted first with hexanes to remove a-pinene and then with 25% EtOAc in hexanes to provide 6(R)-methoxy-3(S)-hydroxy-tetrahydro-2H-pyran (major) and 6(S)-methoxy-3(S)-hydroxy-tetrahydro-2H-pyran (minor) as colorless oil.

EXAMPLE 11

Preparation of 1-Benzyloxy-2(S)-hydroxy-4-pentene

Copper(I)cyanide (120 mg, 1.34 mmol) was added to a solution of (R)-(-)-2-(benzyloxymethyl)-oxirane (2.4 g, 14.6 mmol) in dry THF (200 ml). This mixture was cooled to -78°C under argon atmosphere. A solution of vinylmagnesium bromide in THF (26 ml, 1M, 26 mmol) was added slowly to the epoxide solution at -78°C. The reaction mixture was stirred for 5 hours while the reaction temperature was allowed to warm to 0°C. Saturated aqueous $NR_4Cl$ and $NH_4OH$ were added to the reaction mixture till the reaction mixture became clear. Organic layer was separated and the aqueous layer was extracted with EtOAc (3x50 ml). Combined organic layers were dried over $MgSO_4$ and concentrated. The residue was purified by chromatography (EtOAc/Hexane = 1:9) to give 1-benzyloxy-2(S)-hydroxy-4-pentent (2.6 g, 13.5 mmol) NMR ($CDCl_3$): 7.35 (bs, 5H), 5.84 (m, 1H), 5.14 (d, J = 11, 1H), 5.11 (d, J = 9, 1H), 4.57 (s, 2H), 3.90 (m, 1H), 3.53 (dd, J = 3.4, 9.5, 1H), 3.39 (dd, J = 7.4, 9.5, 1H) 2.29 (t, J = 1.23, 2H).

EXAMPLE 12

Preparation of 2(R)-hydroxy-5(R)-iodomethyl tetrahydrofuran

Solid $NaHCO_3$ (3.6 g, 42.9 mmol) was added to a solution of 1-benzyloxy-2-(S)-hydroxy-4-pentene (1.1 g, 5.7 mmol) in dry $CH_3CN$ (25 ml) and the resulting mixture was cooled to 0°C. To this mixture was added a solution of iodine (2.9 g) in dry acetonitrile. Then the reaction mixture was stirred for 3 hours at 0°C. Saturated $NaHCO_3$ (50 ml) was added to the reaction mixture. Solid $NaHSO_3$ was added to the biphasic mixture until the iodine color disappeared. Organic layer was separated and the aqueous layer was extracted with EtOAc (3 x 50 ml). Combined organic layers were dried over $MgSO_4$ and was concentrated. The reside was purified by chromatography (EtOAc/hexane 1:1) to give the 20(S)-hydroxy-5(R)-iodomethyltetrahydrofuran (232.3 mg), a mixture of isomers (171.5 mg) and 2-(R)-hydroxy-5-(R)-iodomethyltetrahydrofuran (663.1 mg). NMR ($CDCl_3$): 2-(S)-hydroxy-5-(R)-iodomethyltetrahydrofuran: 4.52 (m, 1H), 4.03 (m, 1H), 3.98 (dd, J = 10.8, 1.5, 1H), 3.81 (dd, J = 4.1, 10.8 1H), 3.44 (dd, J = 10, 6.5, 1H), 3.36 (dd, J = 10, 5.3, 1H), 2.36 (m, 1H), 1.84 (m, 1H).
2-(R)-hydroxy-5-(R)-iodomethyltetrahydrofuran:
4.58 (bs, 1H), 4.22 (m, 1H), 4.09 (dd, J = 9.9, 4.1, 1H), 3.30 (d, J = 5.6, 2H), 2.17 (dd, J = 13.3, 5.8, 1H), 1.81 (m, 1H).

EXAMPLE 13

Preparation of 2(RS)-3-phenylpropyl-3(SR)-hydroxy-tetrahydro-2H-pyran

To a solution of 3.0 g of freshly distilled dihydropyran and 0.5 ml of TMEDA at -78°C was added 22.0 ml of a 1.6 m solution of nBuLi in hexane over a period of 15 min. The resulting mixture was stirred at -78° to room temperature for 12h. A white precipitate of the lithium salt of dihydropyran was formed after this period. The mixture was cooled to -78°C and 10 ml of dry THF followed by 10.3 g of phenylpropyl iodide in 5 ml of THF was added over a period of 10 min. The resulting reaction mixture was stirred for 30 min at -78°C and then it was slowly allowed to warm to -15°C. The reaction was quenched at -15°C with saturated solution of $NaHCO_3$ and was extracted thoroughly with ether (2x100ml). The combined extracts were dried over anhydrous $K_2CO_3$ and was used immediately for next reaction.

To the above solution of alkylated dihydropyran in 100 ml of THF-ether (1:1) was added 36.5 ml of a 1 M solution of borane in THF at -30°C. The reaction mixture was stirred at -30°C to room temperature for 12h. After this period, the reaction mixture was quenched at room temperature by adding 6.5 ml of 3N NaOH solution followed by dropwise addition of 4 ml of 30% hydrogen peroxide solution. The resulting mixture was stirred for an additional 2h and 25 ml of brine solution was added to it. The layers were separated and the aqueous phase was extracted with ether (2x50 ml). The combined extracts were dried over anhydrous $Na_2SO_4$ and concentrated under reduced pressure to give an oily residue. The crude product was chromatographed over silica gel (ethyl acetate/hexanes) to provide 5.3 g of the title product as an oil.

EXAMPLE 14

Preparation of 2(R)-Benzyloxymethyl-3(S)-hydroxy tetrahydro-2H-pyran

Step A: Preparation of 2(R)-Benzyloxymethyl-3(S)-hydroxy tetrahydro-2H-pyran

2-(R)-Benzyloxymethyl-3-(S)-methyloxymethyltetrahydro-2H-pyran 0.130 g was dissolved in 5 mL of dry $CH_2Cl_2$ and thiophenol 0.082 g was added followed by 0.073 mL of $BF_3$ $Et_2O$. After stirring for 2 hours, 5 mL of saturated aqueous $NaHCO_3$ was added and the mixture was stirred for 1 hour. The mixture was diluted with 10 mL $CH_2Cl_2$, the layers were separated, and the organic layer was washed with 5% aqueous NaOH (2x5 mL) and brine and dried over anhydrous $Na_2SO_4$. Filtration and evaporation of the solvent provided a residue which was flash chromatographed over silica gel (50% ethyl acetate/hexanes) to afford the title compound as a colorless oil.

EXAMPLE 15

Preparation of 2-Amino-3-methyl cyclopentanol

Step A: Preparation of 2-[N-(1',2'-di-tert-butoxycarbonyl)-hydrazino]-3-methylcyclopentanone:

To a stirred suspension of CuCl, 0.297 g, in 10 ml anhydrous ether cooled to -60°C was added 0.74 ml of tri-n-butylphosphine. The yellow solution was cooled to -73°C, and 4.2 ml of 3M methylmagnesium chloride in THF was added at a rate so that the internal temperature did not exceed -60°C. The cuprate was stirred for 20 minutes at -78°C, forming a dark mustard colored solution. Cyclopentenone, 1.0 ml, was added dropwise keeping temperature below -70°C. After 1.5 hours at -78°C, 2.87 g of di-tert-butyl azodicarboxylate in 13 ml anhydrous THF added slowly over 45 minutes into the reaction mixture. The resulting dark brown mixture was stirred for 2 hours. The reaction was quenched by addition of 1:9 concentrated $NH_4OH$:sat'd $NH_4Cl$, allowed to warm to room temperature overnight, and diluted with ether. The ethereal layer was washed with sat'd $NH_4Cl$, sat'd brine, dried over $Na_2SO_4$ and concentrated under reduced pressure. Purification of the yellow-orange oily residue by chromatography using 20% ethyl acetate in hexane gave 0.78 g, of product as a white crystalline solid, mp 100-101.5°C.

Step B: Preparation of 2-[N-(1',2'-di-tert-butoxycarbonyl)-hydrazine]-3-methylcyclopentanol:

To a stirred solution of the product of Step A, 1.1 g, in anhydrous THF cooled to -78°C was added 10 mL of a 1M solution of lithium tri-sec-butylborohydride in THF. After 4 hours at -78°C the reaction was quenched by dropwise addition of 10 mL of 1N NaOH and 10 mL of 30% hydrogen peroxide. The resulting mixture was allowed to warm to room temperature overnight, and extracted with 3 x 75 mL of ether. The combined organic layers were washed with brine, dried over $MgSO_4$ and concentrated under reduced pressure. Chromatography of the oily residue with 20% ethyl acetate in hexane gave, after recrystallization from ether/hexane 1.5 g of the product as a white solid, mp 146°C.

Step C: Preparation of 2-Amino-3-methyl cyclopentanol

To a stirred solution of the product of Step B, 1.494 g, in 5 mL of $CH_2Cl_2$ cooled to -20°C was added 10 mL of trifluoroacetic acid. The resulting brown mixture was allowed to warm to room temperature overnight and concentrated to dryness under reduced pressure. The brown residual oil, 1.7 g, was dissolved in 104 mL of ethanol and 26 mL of acetic acid containing 240 mg of platinum oxide and shaken under 55 psi of hydrogen for 2.5 hours. The catalyst was filtered off and the solution was concentrated under reduced pressure. Chromatography with 45/9/1 $CHCl_3$/MeOH/conc. $NH_4OH$ gave. after trituration with 1/5 $CHCl_3$/$Et_2O$, 0.75 g of a white amorphous solid, m.p. 109-116°C.
$^1H$ NMR (300 MHz, $CDCl_3$) $\delta$ 3.92-3.96 (m, 1H), 2.59-2.64 (m, 1H), 2.15 (brs, 3H), 1.92 (m, 2H), 1.65 (m, 2H), 1.12 (m, 1H), 1.04 (d, J=6.6 Hz, 3H).

EXAMPLE 16

Preparation of cis-4-aminothiochroman-4-ol

Step A: Preparation of 3-hydroxy thiochroman-4-one dimethylacetal:

A solution of thiochromanone (3.3 g) in 100 mL of absolute methanol was added to a stirred solution of potassium hydroxide (3.4 g ) in 60 mL of methanol over a period of 10 minutes at 0°C. The stirring was continued and iodobenzene diacetate (7.2 g) was added in portions during a 10 minute period. The resulting mixture was then stirred for an additional hour at 0°C and then overnight at room temperature. The reaction mixture was concentrated under reduced pressure and partitioned between 120 mL of saturated potassium carbonate and ether (3 x 100 mL). The combined ether extracts were dried over anhydrous sodium sulfate and concentrated under reduced pressure. The product was an oil (3.6 g).

Step B: Preparation of 3-hydroxy thiochroman-4-one:

To a stirred solution of the product of Step A (3.6 g) in mL of ethanol was added 30 mL of aqueous 3N hydrochloric acid. After stirring for 45 minutes, 50 mL of water was added and the mixture stirred for an additional 45 minutes. The product was isolated by concentrating the mixture to a minimum volume and extracting with ether (3 x 100 mL). The combined ether extracts were washed with aqueous sodium bicarbonate, dried over anhydrous sodium sulfate and concentrated under reduced pressure. The product (2.7 g) was obtained as a foam.

Step C: Preparation of O-benzyl 3-hydroxy thiochroman-4-one oxime ether:

To a stirred solution of the product of Step B (840 mg) in mL of pyridine at 0°C was added 840 mg of benzyloxyamine hydrochloride. The resulting mixture was allowed to warm to room temperature for 12 hours. The solvents were removed under pressure and the residue partitioned between 10 mL of aqueous sodium bicarbonate and ethyl acetate (3 x 100 mL). The combined extracts were dried over anhydrous sodium sulfate and concentrated under reduced pressure. Chromatography of the residue gave a colorless oil.

Step D: Preparation of cis-4-aminothiochroman-3-ol:

To a stirred solution of 1.2 g of O-benzyl oxime in 10 mL dry tetrahydrofuran at 0°C was added 12 mL of a 1 M solution of borane in THF such a rate that the temperature did not exceed 5°C. The resulting mixture was allowed to warm to room temperature for 12 hours. The solvents were removed under reduced pressure and the residue diluted with 5 mL of 10% aqueous potassium hydroxide. The mixture was stirred at reflux for 1 hour, cooled to room temperature, and extracted with ether (3 x 100 mL). The combined extracts were dried over anhydrous sodium sulfate and concentrated under reduced pressure. Chromatography of this crude product gave 615 mg of product as a pale yellow solid: mp 86-88°C; cis/trans ratio 14:1 by:

$^1$H NMR: (300 MHz) δ 7.1 (m, 4H), 4.12 (m, 1H), 3.9 (m, 1H), 3.1-2.85 (m, 2H), 2.3 (br s, 3H).

EXAMPLE 17

Preparation of 4-amino-isothiochromane

Step A: Preparation of O-methyl isothiochroman-4-one oxime:

To a stirred solution of 1.08 g (6.57 mmol) of 1H-2-benzothiopyran-4(3H)-one (isothiochroman-4-one) in 10 mL of dry pyridine was added 0.55 g (6.57 mmol) of methoxylamine hydrochloride. The solution was stirred under an argon atmosphere for 2 hours, after which the solvent was removed *in vacuo* and the residue partitioned between EtOAc and $H_2O$. The aqueous phase was extracted twice more with EtOAc and the combined EtOAc layers were washed with 3 x 15 mL of 1N HCl, then brine solution. After drying over $Na_2SO_4$ the solvent was removed, leaving 1.13 g of a red-brown oil. Chromatography on silica gel (25% EtOAc in hexane) gave 0.880 g of the title compound as colorless needles.

$^1$H NMR ($CDCl_3$) δ 3.71 (s, 2H), 3.82 (s, 2H), 4.02 (s, 3H), 7.12 (m, 1H), 7.25 (m, 2H), 7.98 (m, 1H).

Step B: Preparation of 4-amino-isothiochromane:

38.9 (0.21 mmol) of the product of Step 4 was dissolved in 300 mL of freshly distilled THF and the solution was added dropwise at 0°C to 604 mL of a 1M borane-THF solution. After 30 minutes at 0°C, the reaction mixture was allowed to warm to 25°C and stirring was continued for 36 hours at ambient temperature, or until completed by TLC (25/75 EtOAc/hexane). The mixture was cooled to 0°C and quenched by the dropwise addition of abs. $CH_3OH$. After stirring for 1 hour the mixture was concentrated, then treated with 150 mL of 3N HCl and stirring was continued for 3 hours. The mixture was extracted with ether to remove unreacted starting material. The aqueous phase was basified with 40% NaOH to pH 10 and then extracted with 3 x 200 mL EtOAc. The combined EtOAc layers were washed with $H_2O$, brine; dried ($Na_2SO_4$). Removal of the drying agent and evaporation of the solvent left 28.9 g of pure amine as a deep red oil.

$^1H$ NMR ($CDCl_3$) δ 1.86 (br s, 2H), 2.79 (ddd, J=1.4, 5, 13, H3, 1H), 3.14 (dd, 3=3.6, 13, H3, 1H), 3.61 (d, J=16, H3, 1H), 3.92 (d, J=16, H3, 1H), 4.10 (t, J=4.2, H3, 1H), 7.1-7.4 (m, 4H).

EXAMPLE 18

Preparation of N-((2R)-hydroxy-1(S)-indanyl)-5(S)-(3(S)-tetrahydrofuranyloxycarbonylamino)-4(S)-hydroxy-2-(R)-phenylmethyl-6-cyclohexylhexanamide

Step A: Preparation of N-((2R)-hydroxy-1(S)-indanyl)-5(S)-(1,1-dimethylethyloxycarbonylamino)-4(S) -t-butyldimethylsilyloxy-2(R)-phenylmethyl-6-cyclohexylhexanamide

To a solution of 2.65 g (4.87 mmol) of N'-(1,1-dimethylethoxycarbonyl)-5(S)-amino-4(S)-(1',1'-dimethylethyl-1,1-dimethylsilyloxy)-6-cyclohexyl-2(R)-phenylmethyl-hexanoic acid (synthesized by the methods of Example 2, steps A-D), 776 mg (5.2 mmol) of 1-(S)-amino-2(R)-hydroxyindane, 997 mg (5.2 mmol) of EDC, and 703 mg (5.2 mmol) of 1-hydroxybenzotriazole hydrate in 50 mL of DMF was added 1.45 mL (10.4 mmol) of triethylamine under Ar. After stirring for 22 h at room temperature, an additional 500 μL of triethylamine, 200 mg of 1-(S)-amino-2(R)-hydroxyindane, and 200 mg of EDC were added and stirred for an additional 4 h, then heated to 30° overnight. The solvents were removed at reduced pressure and the residue partitioned between cold 1M citric acid and EtOAc. The aqueous layer was extracted with EtOAc, the combined organic layers were washed with 10% $Na_2CO_3$, brine, dried over $Na_2SO_4$, and the solvents removed to give 4 g of an oil which was chromatographed on 300 g of fine $SiO_2$ using 1:3 ethyl acetate-hexane to afford 1.66 g of the title compound as a colorless foam:

$^1H$ NMR ($CDCl_3$) δ 0.1 (s, 3H), 0.12 (s, 3H), 0.85-1.1(m, 2H), 0.93 (s, 9H), 1.1-2.1 (m, 10H), 1.36 (s, 9H), 2.7-3.1 (m, 6H), 3.75-3.9 (m, 2H), 4.28-4.32 (m, 1H), 4.66 (d, J= 10Hz, 1H), 5.29-5.35 (m, 1H), 6.05 (d, J= 9Hz, 1H, 7.12-7.40 (m, 9H).

Step B: Preparation of N-((2R)-hydroxy-1(S)-indanyl)-5(S)-(3(S)-tetrahydrofuranyloxycarbonylamino) -4(S)-hydroxy-2(R)-phenylmethyl-6-cyclohexyl-hexanamide

To a stirred solution of 1.66 g (3.11 mmol) of the product from Step A in 56 mL of acetonitrile at 0° was added 1.31 mL of 9.9 M ethanolic HCl in two portions. The cold bath was removed and stirring continued for 5.3 h. The solvents were removed at reduced pressure, and the residue partitioned between 125 mL of $CHCl_3$ and 10% $Na_2CO_3$. The organic layer was dried over $Na_2SO_4$ and the solvents removed to give a colorless glass which was dissolved in 20 mL of DMF and treated with 756 mg (3.3 mmol) of 3(S)-tetrahydrofuranyloxy-N-hydroxysuccinimide carbonate and 460 μL (3.3 mmol) of triethylamine under Ar. After stirring at room temperature overnight, the solvents were removed at reduced pressure and the residue partitioned between 9:1 $CHCl_3$-$CH_3OH$ and 1M citric acid. The aqueous layer was extracted with 9:1 $CHCl_3$-$CH_30H$, the combined organic layers washed with 10% $Na_2CO_3$, dried over $Na_2SO_4$ and the solvents removed to give a colorless solid. This material was chromatographed on 200 g of fine $SiO_2$ using 98.5:1.5 to 98:2 $CHCl_3$-$CH_3OH$ to give 0.8 g of a gel-like solid which was crystallized from 80 mL of acetonitrile to provide 320 mg of a colorless solid. Recrystallization from 17 mL of acetonitrile afforded 196 mg of the title compound as a colorless solid: mp = 183.5-185.5°C

$^1H$ NMR ($CDCl_3$) δ 0.8-1.05 (m, 2H), 1.05-1.55(m,6H), 1.55-2.10 (m,10H), 2.77-3.10 (m, 5H), 3.55-3.72 (m, 2H), 3.72-3.93 (m, 4H), 4.26 (t, J= 5 Hz, 1H), 5.12-5.20 (m, 1H), 5.25 (d, 5H, 1H), 6.55 (d, J= 10 Hz, 1H), 7.08-7.37 (m, 9H).

| | | | |
|---|---|---|---|
| Anal. Calc'd for $C_{33}H_{44}N_2O_6$ | C 70.19 | H 7.85 | N 4.96 |
| Found | C 69.94 | H 7.69 | N 5.19 |

EXAMPLE 19

Preparation of N-((2R)-(L-ornithinoyloxy)-1(S)-indanyl)-5(S)-(3(S)-tetrahydrofuranyloxycarbonylamino) -4(S)-hydroxy-2(R)-phenylmethyl-6-cyclohexylhexanamide

Step A: Preparation of N-((2R)-(N-α-N-δ-bis-benzyloxycarbonyl-L-ornithinoyloxy)-1(S)-indanyl)-5(S)-(3(S)-tetrahydrofuranyloxycarbonylamino) -4(S)-hydroxy-2(R)-phenylmethyl-6-cyclohexyl hexanamide

To a stirred solution of 466 mg (0.825 mmol) of the product from Example 18, 331 mg (0.825 mmol) of N-α-N-δ-bis-benzyloxycarbonyl-L-ornithine, and 20 mg (0.165 mmol) of dimethylaminopyridine in 16 mL of 1:1 THF-$CH_2Cl_2$ under Ar at room temperature was added 171 mg (0.825 mmol) of dicyclohexylcarbodiimide as a solid in one portion. After 22 h, the precipitate was filtered and washed with 1:1 THF-$CH_2Cl_2$. The filtrate was diluted with $CHCl_3$ and washed with cold 1M citric acid, saturated $NaHCO_3$, dried over $Na_2SO_4$ and the solvents removed to give 0.9 g of a colorless oil which was chromatographed on 80 g of fine $SiO_2$ using 99:1 to 97.5:2.5 $CHCl_3$-$CH_3OH$. This afforded 430 mg of a colorless solid:
NMR (DMSO-$d_6$)) δ 0.7-0.97 (m, 2H), 1.0-1.90(m, 18H), 1.97-2.08(m, 1H), 2.53-2.60(m, 1H), 2.60-3.0(m, 5H), 3.20-3.3(m, 1H), 3.4-3.53(m, 2H), 3.55-380(m, 4H), 4.0-4.16(m, 1H), 4.3-4.45(m, 1H), 4.81(d, J = 12 Hz, 1H), 4.93(d, J = 12Hz, 1H), 4.98(s, 2H), 4.99-5.05(m, 1H), 5.42(t, J = 5Hz, 1H), 5.59(dd, J = 9Hz, 5.3 Hz, 1H), 6.63(d, J = 9Hz, 1H), 7.05-7.40(m, 20H), 7.57 (d,8Hz, 1H), 7.90(d, J = 9Hz, 1H).

Step B: Preparation of N-((2R)-(L-ornithinoyloxy)-1(S)-indanyl)-5(S)-(3(S)-tetrahydrofuranyloxy carbonylamino)-4(S)-hydroxy-2(R)-phenylmethyl -6-cyclohexylhexanamide

To a stirred suspension of 430 mg (0.454 mmol) of the product from Step A in 39 mL of ethanol, 8 mL of THF, and 0.18 mL of acetic acid was added 454 μL of aqueous 2M HC1 and 180 mg of 10% Pd on carbon. The flask was evacuated and fitted with a hydrogen balloon. After stirring for 5 h at room temperature, the mixture was filtered through a 2μ glass fiber filter. The catalyst was washed with 95% ethanol and the combined filtrates were concentrated to a small volume. This residue was lyophilized from 40 mL of 95:5 $H_2O$-HOAc to provide the title compound as colorless solid:
$^1$H NMR (DMSO-$d_6$) δ 0.7-0.95(m, 2H), 1.02-1.34(m, 5H), 1.50-1.90(m, 9H), 1.97-2.10(m, 1H), 2.52-2.60(m, 1H), 2.65(br t, 1H), 2.85-2.95(m, 1H), 3.0(br d, J = 17 Hz, 1H), 3.13-3.25(m, 2H), 3.42-3.57(m, 2H), 3.59-3.80(m, 4H), 3.93-4.0(m, 1H), 4.51-4.57(m, 1H), 5.03-5.10(m, 1H), 5.40-5.46(m, 1H), 5.66(dd, J = 9 Hz, 5 Hz, 1H), 6.67(d, J = 9Hz, 1H), 7.13-7.35(m, 9H), 7.97(br s, 2H), 8.40-8.50(m, 1H), 8.63(br s, 2H).

| Anal. Calc'd for $C_{38}H_{54}N_4O_7$ 2HCl 2.6 $H_2O$ | | | |
|---|---|---|---|
| | C 57.15 | H 7.72 | N 7.01 |
| Found | C 56.79 | H 7.35 | N 6.87 |

EXAMPLE 20

Preparation of N-((2R)-hydroxy-1(S)-indanyl)-5(S)-(3(S)-tetrahydrofuranyloxycarbonylamino)-4(S)-hydroxy-2-(R)-isopropyl-6-cyclohexylhexanamide

The title compound was prepared by the methods of Example 18. The compound (5S,3R,1'S)-3-isopropyl-5-(1'-((1,1-dimethylethyloxycarbonyl)amino)-2'-cyclohexylethyl)-4,5-dihydrofuran-2-(3H)-onewas prepared according to Nishi, T. et al. Chem Lett. __, 1993 (1989). The title compound had the following characteristics:
mp 199-200 °C,
$^1$H NMR (DMSO-$d_6$) δ 0.7-0.95(m, 2H), 0.88(d, J = 7 Hz, 3H), 0.89(d, J = 7 Hz, 3H), 1.05-1.38(m, 7H), 1.55-188(m, 8H), 1.98-2.10(m, 1H), 2.23-2.31(m, 1H), 2.79(d, J = 16 Hz, 1H), 3.02(dd, J = 16 Hz, 4 Hz, 1H), 3.42-3.58(m, 2H), 3.59-3.79(m, 4H), 4.34-4.40(m,1H), 4.51(d, J = 5 Hz, 1H), 5.00(d, J = 4 Hz, 1H),

5.06-5.12(m, 1H), 5.22(dd, J = 8 Hz, 9 Hz, 1H), 6.71(d, J = 9 Hz, 1H), 7.08-7.23(m, 4H), 7.49(d, J = 9 Hz, 1H).

| Anal. Calc'd for $C_{33}H_{44}N_2O_6$ | | | |
|---|---|---|---|
| | C 70.19 | H 7.85 | N 4.96 |
| Found | C 69.94 | H 7.69 | N 5.19 |

Example 21

Preparation of N-((2R)-(L-ornithinoyloxy)-1(S)-indanyl)-5(S)-(3(S)-tetrahydrofuranyloxycarbonylamino)-4(S)-hydroxy-2(R)-isopropyl-6-cyclohexylhexanamide

The title compound was prepared by the methods of Example 19.
$^1$H NMR (DMSO-$d_6$) δ 0.75-0.98(m, 2H), 0.83(d, J = 7 Hz, 3H), 0.86(d, J = 7 Hz, 3H), 1.05-1.38(m, 7H), 1.52-1.88(m, 14H), 1.97-2.09(m, 1H), 2.49-2.57(m, 1H), 2.69(t, J = 6.8 Hz, 2H), 2.99(d, J = 17 Hz, 1H), 3.45-3.59(m, 2H), 3.59-3.79(m, 4H), 3.98(t, J = 6.6 Hz, 1H), 4.52(d, J = 6 Hz, 1H), 5.05-5.11(m, 1H), 5.49(t, J = 5 Hz, 1H), 5.68-5.73(m, 1H), 6.77(d, J = 9 Hz, 1H), 7.13-7.30(m, 4H), 7.75-8.80(br s, 6H), 8.26(d, J = 9 Hz, 1H).

| Anal. Calc'd for $C_{34}H_{54}N_4O_7$ 2.0 HCL 2.0 $H_2O$ | | | |
|---|---|---|---|
| | C 55.20 | H 8.18 | N 7.57 |
| Found | C 54.99 | H 7.99 | N 7.66 |

Assay for Inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30°C for 1 hour. Various concentrations of inhibitor in 1.0 ul DMSO were added to 25 ul of the peptide solution in water. The reaction is initiated by the addition of 15 ul of 0.33 nM protease (0.11 ng) in a solution of 0.133 M Na acetate pH 5.5 and 0.26% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. The compounds of this invention showed $IC_{50}$ values in the range of about 0.1nM - 100μM.

The preferred species had the following activity:

| Compound | $IC_{50}$(nM) |
|---|---|
| A | 0.37 |
| C | 0.04 |

While the foregoing specification teaches the principles of the present invention, with examples provided for the purpose of illustration, it will be understood that the practice of the invention emcompasses all of the usual variations, adaptations, or modifications, as come within the scope of the following claims and its equivalents.

**Claims**

**1.** A compound of formula I

A-G-J    I,

wherein

A is:

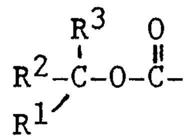

wherein $R^1$, is

a) H, or

b) $C_{1-6}$ alkyl unsubstituted or substituted with one or more of

i) OH

ii) alkoxy; and

$R^2$ and $R^3$ are joined to form a 5-7 membered heterocyclic ring, or 7-10 membered bicyclic heterocyclic ring, saturated or unsaturated, unsubstituted or substituted with one or more of $R^4$ wherein

$R^4$ is

$R^5$-$(CH_2)_n$-, $R^5(CH_2)_nO$-,
$R^5$-$(CH_2)_nS$-, $R^5(CH_2)_nS(O)$-, or
$R^5$-$(CH_2)SO_2$- wherein

$R^5$ is

aryl, heterocycle,
$C_{1-4}$ alkyl or H and n is 0-5.

G is

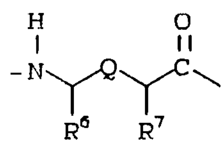

or

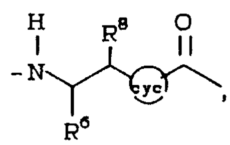

,

$R^6$ and $R^7$ are independently,

a) $-(CH_2)_n-R^9$

b) $C_{1-4}$alkenyl $-R^9$;

wherein n is 0-5

$R^9$ is

a) hydrogen;

b) $C_{1-4}$ alkyl;

C) $C_5-C_{10}$ cycloalkyl, unsubstituted or substituted one or more times with hydroxy;

d) $C_6$-$C_{10}$ aryl, unsubstituted or substituted one or more times with;

i) halo,

ii) $C_{1-4}$alkyl, unsubstituted or substituted once with hydroxy,

iii) $C_{1-3}$ alkoxy, unsubstituted or substituted once with hydroxy,

iv) hydroxy;

e) monocyclic or bicyclic heterocycle containing from 1 to 3 heteroatoms chosen from N,O,S and which is unsubstituted or substituted with one or more of

i) halo,

ii) $C_{1-4}$ alkyl,

iii) $C_{1-3}$ alkoxy;

$R^8$ is -OH or $-NHR^{10}$, wherein $R^{10}$ is -H,

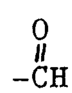

,

or -$C_{1-4}$- alkyl;

Q is

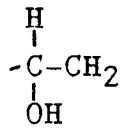 ; 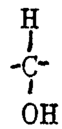 ; or 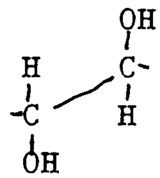

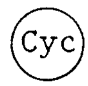 is

1) $C_{3-7}$ cycloalkyl either unsubstituted or substituted with one or more of

a) $C_{1-4}$ alkyl,

b) hydroxy,

c) -$NR_2$, wherein R is H or $C_{1-4}$ alkyl;

d) -COOR,

e) -CONHR,

f) -$NHSO_2R$,

g)

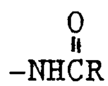 ,

h) aryl,

i) aryl substituted with $C_{1-4}$alkyl,

j) heterocycle, or

k) heterocycle substituted with $C_{1-4}$alkyl;

J is

a) $NHR^{11}$ wherein $R^{11}$ is a 5-7 membered heterocyclic ring or, 7-10 membered bicyclic heterocyclic ring which is saturated or unsaturated, the ring or rings being unsubstituted or substituted with one or more of

i) halo,

ii) -OR, wherein R is H, $C_{1-4}$alkyl, or $C_{1-4}$alkenyl,

iii)

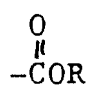 ,

iv)

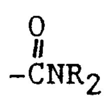 ,

v) -$CH_2NR_2$,

vi) -$SO_2NR_2$,

vii) -$NR_2$,

viii)

$$-NH\overset{\overset{O}{\|}}{C}R,$$

xi) $C_{1-4}$ alkyl,
x) phenyl
xi) $-CF_3$,
xii)

$$\overset{R}{-N}-SO_2R,$$

xiii) phenyl $C_{1-4}$ alkyl,
xiv) $-OP(O)(OR_x)_2$ wherein $R_x$ is H or aryl,
xv)

$$-O-\overset{\overset{O}{\|}}{C}-C_{1-4}alkyl$$

substituted with one or more of amine or quaternary amine, or $-OP(O)(OR_x)_2$, or $-O[(CH_2)_mO]_n-R$, wherein m is 2-5, n is 0-5; or
xvi)

$$-O-\overset{\overset{O}{\|}}{C}-O-[(CH_2)_mO]_n-R;$$

b) A 5 to 7 membered carbocyclic or 7-10 membered bicyclic carbocyclic ring which is either saturated or unsaturated, such as cyclopentane, cyclohexane, indane, norbornane, or naphthalene, the carbocyclic ring being unsubstituted or substituted with one or more of
i) halo,
ii) -OR, or $-CH_2OR$, wherein R is H or $C_{1-4}$ alkyl,
iii)

$$-\overset{\overset{O}{\|}}{C}OR^{12},$$

wherein $R^{12}$ is H, $-(CH_2)_n-NR_2$, $C_{1-16}$alkyl, pyridine, $-(CH_2)_nNR-(CH_2)_n-NR_2$,

$$-(CH_2)_n-\overset{\overset{O}{\|}}{C}-OR,$$

$-[(CH_2)_mO]_n-R$, quinuclidiniumyl substituted with R, piperazine-$C_{1-4}$alkyl-benzyl substituted once or more with R, or morpholinoalkylbenzyl,
iv)

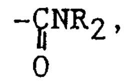

v) $-CH_2NR_2$,
vi) $-SO_2NR_2$,
vii) $-NR_2$,
viii)

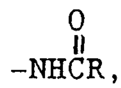

xi) $C_{1-4}$ alkyl,
x) phenyl,
xi) $-CF_3$,
xii)

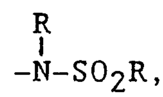

xiii) $-OP(O)(OR_x)_2$ wherein $R_x$ is H or aryl,
xiv)

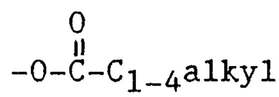

substituted with one or more of amine or quaternary amine, $-OP(O)(OR_x)_2$, or $-O-[(CH_2)_mO]_n-R$, or
xv)

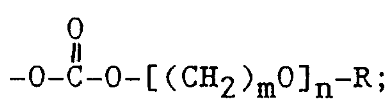

or pharmaceutically acceptable salts or esters thereof.

**2.** A compound according to Claim 1, wherein:
A is tetrahydrofuranyloxycarbonyl or tetrahydropyranyloxycarbonyl, unsubstituted or substituted with $R_4$; and

G is 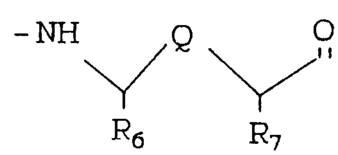

**3.** A compound according to Claim 2, wherein:
Q is

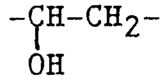 .

4. A compound according to Claim 3, wherein J is NH-$R^{11}$ wherein $R^{11}$ is a 7 to 10 membered bicyclic carbocyclic or heterocyclic ring which is either saturated or unsaturated and is substituted with hydroxyl.

5. A compound according to Claim 4, wherein $R^{11}$ is indanyl substituted once or twice with OH.

6. A compound, which is:

N-(2(R)-hydroxy-1(S)-indanyl)-5(S)-(3(S)-tetrahydrofuranyloxycarbonylamino)-4(S)-hydroxy-6-phenyl-2-(R)-(4-(2-hydroxyethoxy)phenyl)methyl) hexanamide,

N-(2(R)-hydroxy-1(S)-indanyl)-5(S)-(3(S)-tetrahydrofuranyloxycarbonylamino)-4(S)-hydroxy-6-phenyl-2-(R)-phenylmethyl hexanamide,

N-(2(R)-hydroxy-1(S)-indanyl)-5(S)-(3(S)-tetrahydrofuranyloxycarbonylamino)-4(S)-hydroxy-6-phenyl-2-(R)-methyl hexanamide,

N-(2(R)-hydroxy-1(S)-indanyl)-5(S)-(3(S)-tetrahydrofuranyloxycarbonylamino)-4(S)-hydroxy-6-phenyl-2-(R)-(trans-3-(phenyl)prop-2-en-1-yl) hexanamide,

N-(2(R)-hydroxy-1(S)-indanyl)-5(S)-(3(S)-tetrahydrofuranyloxycarbonylamino)-4(S)-hydroxy-6-phenyl-2-(R)-ethyl hexanamide,

N-(2(R)-hydroxy-1(S)-indanyl)-S(S)-(3(S)-tetrahydrofuranyloxycarbonylamino)-4(S)-hydroxy-6-phenyl-2-(R)-cyclopropylmethyl hexanamide,

N-(2(R)-hydroxy-1(S)-indanyl)-5(S)-(3(S)-tetrahydrofuranyloxycarbonylamino)-4(S)-hydroxy-6-cyclohexyl-2-(R)-cyclohexylmethyl hexanamide,

N-(2(R)-hydroxy-3(R)-amino-1(S)-indanyl)-5(S)-(3(S)-tetrahydrofuranyloxycarbonylamino)-4(S)-hydroxy-6-cyclohexyl-2(R)-phenylmethyl hexanamide,

N-(2(R)-hydroxy-1(S)-indanyl)-5(S)-(3(S)-tetrahydrofuranyloxycarbonylamino)-4(S)-hydroxy-6-cyclohexyl-2(R)-phenylmethyl hexanamide,

N-(2(R)-hydroxy-1(S)-indanyl)-5(S)-(3(S)-tetrahydrofuranyloxycarbonylamino)-4(S)-hydroxy-6-cyclohexyl-2(R)-(4-(2-hydroxyethoxy)phenyl)methyl hexanamide,

N-(2(R)-hydroxy-1(S)-indanyl)-5(S)-(3(S)-tetrahydrofuranyloxycarbonylamino)-4(S)-hydroxy-6-cyclohexyl-2(R)-(1-methylethyl) hexanamide,

N-(2(R)-hydroxy-1(S)-indanyl)-5(S)-(3(S)-tetrahydrofuranyloxycarbonylamino)-4(S)-hydroxy-7-methyl-2-(R)-phenylmethyl octanamide,

N-(2-(R)-hydroxy-5(R)-methyl-1(S)-cyclopentyl)-5(S)-(3(S)-tetrahydrofuranyloxycarbonylamino)-4(S)-hydroxy-6-phenyl-2(R)-(4-(2-hydroxyethoxy)phenyl)-methyl hexanamide,

N-(3,4-dihydro-2,2-dioxo-1H-4(S)-benzo-2-thiopyranyl)-5(S)-(3(S)-tetrahydrofuranyloxycarbonylamino)-4-(S)-hydroxy-6-phenyl-2(R)-phenylmethyl hexanamide,

N-(3,4-dihydro-1H-4(S)-benzo-2-thiopyranyl)-5(S)-(3(S)-tetrahydrofuranyloxycarbonylamino)-4(S)-hydroxy-6-phenyl-2(R)-(3-phenyl-prop-2-en-1-yl)methyl hexanamide,

N-(3,4-dihydro-2(R,S)-oxo-1H-4(S)-benzo-2-thiopyranyl)-5(S)-(3(S)-tetrahydrofuranyloxycarbonylamino)-4(S)-hydroxy-6-phenyl-2(R)-(3-phenylprop-2-en-1-yl)methyl hexanamide,

N-(2(R)-hydroxy-1(S)-indanyl)-5(S)-(3(R,S)-tetrahydropyranyloxycarbonylamino)-4(S)-hydroxy-6-phenyl-2(R)-(3-phenyl-prop-2-en-1-yl)methyl hexanamide,

N-(1(R)-amino-2(R)-hydroxy-3(S)-indanyl)-5(S)-(3(S)-tetrahydrofuranyloxycarbonylamino)-4(S)-hydroxy-6-cyclohexyl-2(R)-phenylmethyl hexanamide,

N-(3,4-dihydro-2,2-dioxo-1H-4(S)-benzo-2-thiopyranyl)-5(S)-(3(S)-tetrahydrofuranyloxycarbonylamino)-4-(S)-hydroxy-6-phenyl-2(R)-(3-phenyl-prop-2-en-1-yl)methyl hexanamide,

N-(2(R)-hydroxy-1(S)-indanyl)-5(S)-(3(S)-1,1-dioxotetrahydrothiofuranyloxycarbonylamino)-4(S)-hydroxy-6-phenyl-2(R)-(3-(phenyl)prop-2-en-1-yl)hexamide,

N-(2(R)-hydroxy-1(S)-indanyl)-5(S)-(3(S)-1,1-dioxo-tetrahydrothiopyranyloxycarbonylamino)-4(S)-hydroxy-6-phenyl-2(R)-(3-(phenyl-prop-2-en-1-yl) hexanamide,

N-(2(R)-hydroxy-1(S)-indanyl)-5(S)-(3(S)-tetrahydrofuranyloxycarbonylamino)-4(S)-hydroxy-6-methyl-2-(R)-(1-methylethyl) heptanamide,

N-(2(R)-hydroxy-1(S)-indanyl)-5(S)-(3(S)-tetrahydrofuranyloxycarbonylamino)-4(S)-hydroxy-6-methyl-2-(R)-phenylmethyl heptanamide,

N-(2(R)-hydroxy-1(S)-indanyl)-5(S)-(3(S)-tetrahydrofuranyloxycarbonylamino)-4(S)-hydroxy-6-methyl-2-(R)-(3-phenyl-prop-2-en-1-yl)methyl heptanamide,

N-(2(R)-hydroxy-1(S)-indanyl)-5(S)-(3(S)-tetrahydrofuranyloxycarbonylamino)-4(S)-hydroxy-7-methyl-2-(R)-(1-methylethyl) octanamide,

N-(2(R)-hydroxy-1(S)-indanyl)-5(S)-(3(S)-tetrahydrofuranyloxycarbonylamino)-4(S)-hydroxy-7-methyl-2-(R)-(3-phenyl-prop-2-en-1-yl)methyl octanamide,

N-(2(R)-hydroxy-1(S)-indanyl)-5(S)-(3(R,S)-pyrrolidinyloxycarbonylamino)-4(S)-hydroxy-6-phenyl-2(R)-[4-(2-hydroxyethoxyphenyl)methyl]hexanamide,

N-(2(R)-hydroxy-1(S)-indanyl)-5(S)-(1-benzyloxycarbonyl-3(R,S)-pyrrolidinyloxycarbonylamino)-4(S)-hydroxy-6-phenyl-2(R)-[4-(2-hydroxyethoxyphenyl)-methyl hexanamide,

N-[cis-2(R)-hydroxy-1(S)-indanyl]-5-[3(S)-tetrahydro-2H-pyranyloxycarbonylamino]-4(S)-hydroxy-6-cyclohexyl-2(R)-(3-phenyl-2-propen-1-yl)-hexanamide,

N-[cis-2(S)-hydroxy-1(S)-benzopyranyl]-5-[3(S)-tetrahydrofuranyloxycarbonylamino]-4(S)-hydroxy-2(R)-phenylmethyl pentanamide,

N-[cis-2(S)-hydroxy-1(S)-benzopyranyl]-5-[3(R)-(2-(S)-4-phenylbutyl)tetrahydro-2H-pyranyloxycarbonylamino]-4(S)-hydroxy-2(R)-phenylmethyl pentanamide,

N-[cis-2(S)-hydroxy-1(S)-benzopyranyl]-5-[3(S)-(2-(R)-4-phenylbutyl)tetrahydro-2H-pyranyloxycarbonylamino]-4(S)-hydroxy-2(R)-phenylmethyl pentanamide,

N-[cis-2(S)-hydroxy-1(S)-benzopyranyl]-5-[3(R)-(2-(S)-3-phenylpropyl)tetrahydro-2H-pyranyloxycarbonylamino]-4(S)-hydroxy-2(R)-phenylmethyl pentanamide,

N-[cis-2(S)-hydroxy-1(S)-benzopyranyl]-5-[3(S)-(2-(R)-3-phenylpropyl)tetrahydro-2H-pyranyloxycarbonylamino]-4(S)-hydroxy-2(R)-phenylmethyl pentanamide,

N-[cis-2(R)-hydroxy-1(S)-indanyl]-5(S)-[3'(S)-tetrahydrofuranyloxycarbonylamino]-4(S)-hydroxy-6-cyclohexyl-2(R)-(3-phenyl-2-propen-1-yl)-hexanamide,

N-[cis-2(R)-hydroxy-1(S)-indanyl]-5(S)-[3(S)-tetrahydropyranyloxycarbonylamino]-4(S)-hydroxy-6-cyclohexyl-2(R)-(3-phenyl-2-propen-1-yl)-hexanamide,

N-[cis-2(R)-hydroxy-1(S)-indanyl]-5(S)-[3(SR)-tetrahydrothiofuranyloxycarbonylamino]-4(s)-hydroxy-6-cyclohexyl-2(R)-(3-phenyl-2-propen-1-yl)-hexanamide,

N-[cis-2(R)-hydroxy-1(S)-indanyl]-5(S)-[3(SR)-1-oxo tetrahydrothiofuranyloxycarbonylamino]-4(S)-hydroxy-6-cyclohexyl-2(R)-(3-phenyl-2-propen-1-yl)-hexanamide,

N-[cis-2(R)-hydroxy-1(S)-indanyl]-5(S)-[3(SR)-1,1-dioxo-tetrahydrothiofuranyloxycarbonylamino] -4(S)-hydroxy-6-cyclohexyl-2(R)-(3-phenyl-2-propen-1-yl)-hexanamide,

N-[cis-2(R)-hydroxy-1(S)-indanyl]-5(S)-[4'(SR)-tetrahydrothiopyranyloxycarbonylamino]-4(S)-hydroxy-6-cyclohexyl-2(R)-(3-phenyl-2-propen-1-yl)-hexanamide,

N-[cis-2(R)-hydroxy-1(S)-indanyl]-5(S)-[4(SR)-1,1-dioxo- tetrahydrothiopyranylcarbonylamino]-4(S)-hydroxy-6-cyclohexyl-2(R)-(3-phenyl-2,-propen-1'-yl)-hexanamide,

N-[cis-2(R)-hydroxy-1(S)-indanyl]-5(S)-[6(R)-1-oxo methoxy-3(S)-tetrahydropyranylcarbonylamino]-4(S)-hydroxy-6-cyclohexyl-2(R)-(3-phenyl-2-propen-1-yl)-hexanamide,

N-[cis-2(R)-hydroxy-1(S)-indanyl]-5(S)-[6(S)-methoxy-3(S)-tetrahydropyranyloxycarbonylamino]-4(S)-hydroxy-6-cyclohexyl-2(R)-(3-phenyl-2-propen-1-yl)-hexanamide,

N-[cis-2(R)-hydroxy-1(5)-indanyl]-5(S)-[5(S)-iodomethyl-3(S)-tetrahydrofuranyloxycarbonylamino]-4(S)-hydroxy-6-cyclohexyl-2(R)-(3-phenyl-2-propen-1-yl)-hexanamide,

N-[(2R)-hydroxy-1(S)-indanyl]-5(S)-(3(S)-tetrahydrofuranyloxycarbonylamino)-4(S)-hydroxy-6-cyclohexyl-2(R)-isopropylhexanamide,

N-[(2R)-hydroxy-1(S)-indanyl ornithine ester]-5(S)-(3(S)-tetrahydrofuranyloxycarbonylamino)-4(S)-hydroxy-6-cyclohexyl-2(R)-isopropyl hexanamide,

N-[(2R)-hydroxy-1(S)-indanyl]-5(S)-(3(S)-tetrahydrofuranyloxycarbonylamino)-4(S)-hydroxy-6-cyclohexyl-2(R)-phenylmethyl hexanamide, or

N-[(2R)-hydroxy-1(S)-indanyl ornithine ester]-5(S)-(3(S)-tetrahydrofuranyloxycarbonylamino)-4(S)-hydroxy-6-cyclohexyl-2(R)-phenyl methyl hexanamide,

or pharmaceutically acceptable salts or esters thereof.

**7.** A pharmaceutical composition comprising a compound as claimed in any of Claims 1-6, and a pharmaceutically acceptable carrier.

**8.** The use of a compound as claimed in any one of Claims 1-6 for the manufacture of a medicament for inhibiting HIV protease.

**9.** The use of a compound as claimed in any one of Claims 1-6 for the manufacture of a medicament for preventing infection of HIV, or treating infection by HIV or treating AIDS or ARC.

European Patent Office

# PARTIAL EUROPEAN SEARCH REPORT

which under Rule 45 of the European Patent Convention shall be considered, for the purposes of subsequent proceedings, as the European search report

Application Number
EP 92 20 2447

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | EP-A-0 434 365 (MERCK AND CO. INC.)<br>* pages 23, 28-31, 45; claims 1-4, 13, 15, 17, 19, 21, 23 *<br>--- | 1-4,7-9 | C07D207/12<br>C07D307/20<br>C07D309/10<br>C07D333/32<br>C07D333/48<br>C07D335/02<br>C07D407/12<br>C07D409/12<br>A61K31/35<br>A61K31/38<br>A61K31/41 |
| A,D | EP-A-0 337 714 (MERCK AND CO. INC.)<br>* pages 16-19; claims 1, 14, 27, 29, 31, 33, 35, 37 *<br>--- | 1-4,7,9 | |
| A,P | WO-A-9 118 866 (DU PONT MERCK PHARMACEUTICAL CO.)<br>* pages 67-70, 115-126; claims 1-3, 16-18, 31-33 *<br>--- | 1,7-9 | |
| A,D | US-A-4 661 473 (J.S. BOGER ET AL.)<br>--- | | |
| A,D | EP-A-0 157 409 (MERCK AND CO. INC.)<br>----- | | |

TECHNICAL FIELDS SEARCHED (Int. Cl. 5)

C07D
C07K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely: 5-9
Claims searched incompletely: 1-4
Claims not searched: ---
Reason for the limitation of the search:

see sheet C

| Place of search | Date of completion of the search | Examiner |
|---|---|---|
| BERLIN | 04 DECEMBER 1992 | Christian Hass |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

SHEET C EP 92202447

Only a few of the large number of compounds which are comprised by the formula A-G-J of claim 1 are exemplified in the description. Additionally, the formulation of Claim 1 is such that it does not meet the requirements of Art. 84 EPC (claims shall be clear and concise). Claims 2-4 do not limit the subject matter of Claim 1 such that a complete search could be carried out economically (see Guidelines B 111 3.7). Therefore the search was limited to the subject-matter comprised by Claims 5-9 and to the compounds comprised by the examples and the tables in the description.